# EUROPEAN PATENT SPECIFICATION

(11) **EP 3 044 595 B1**
(45) Date of publication and mention of the grant of the patent: **25.07.2018**
(21) Application number: 14843287.5
(22) Date of filing: 12.09.2014
(51) Int. Cl.: G01N 35/00, B01L 3/00

(54) **DYNAMIC ASSAY SELECTION AND SAMPLE PREPARATION APPARATUS AND METHODS AND MACHINE-READABLE MEDIUMS THEREOF**
DYNAMISCHE TESTAUSWAHL- UND PROBENVORBEREITUNGSVORRICHTUNG SOWIE VERFAHREN DAFÜR UND MASCHINENLESBARE MEDIEN DAFÜR
APPAREIL ET PROCÉDÉS DE SÉLECTION DYNAMIQUE DE DOSAGE ET DE PRÉPARATION D'ÉCHANTILLONS, ET SUPPORTS LISIBLES PAR MACHINE ASSOCIÉS

(30) Priority: 12.09.2013 US 201361876798 P
(43) Date of publication of application: 20.07.2016
(73) Proprietor: Siemens Healthcare Diagnostics Inc., Tarrytown, NY 10591 (US)
(72) Inventor: NATALE, Alfonso, I-21047 Saronno (VA) (IT); FIGUEROA, Israel, Oakland, California 94609 (US)
(74) Representative: Schweitzer, Klaus
(86) International application number: PCT/US2014/055420
(87) International publication number: WO 2015/038910

(56) References cited:
- US-A1- 2004 029 260
- US-A1- 2004 033 610
- US-A1- 2006 051 243
- US-A1- 2012 156 683
- US-A1- 2013 157 273
- US-A1- 2013 157 273

## Description

### RELATED APPLICATIONS

This application claims priority to U.S. Provisional Application Serial Number 61/876,798 entitled "OPEN DYNAMIC ASSAY SELECTION AND SAMPLE PREPARATION" filed on September 12, 2013.

### FIELD

The present invention relates generally to methods and apparatus for preparing test plates for performing assays, such as by using polymerase chain reaction (PCR) testing.

### BACKGROUND

A wide variety of diagnostic instruments (e.g., clinical analyzers or immunoassay Instruments) are used to analyze patient specimens (biological samples). These diagnostic instruments may conduct assays (e.g., immunoassays) using magnetic particles, one or more reagents, buffers, controls, or other additives to identify one or more components (e.g., nucleic acids) in, or characteristics of, a patient sample. Some immunoassay systems may use polymerase chain reaction (PCR), wherein a sample preparation apparatus providing automated sample preparation and extraction technology is used. Once the samples are prepared by the automated sample preparation system, an amplification and detection (AD) device may be used to isolate and purify both DNA and/or RNA from processed eluate from the biological samples.

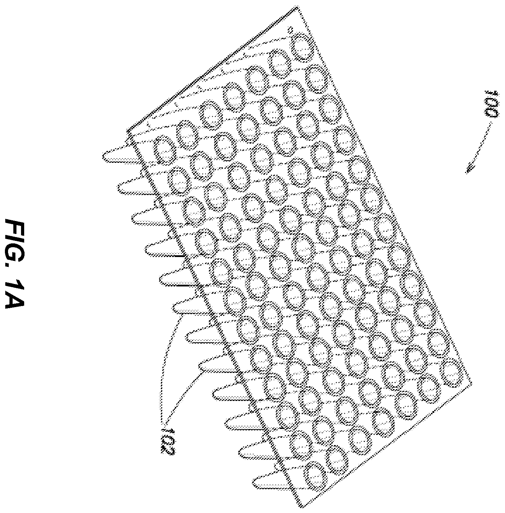

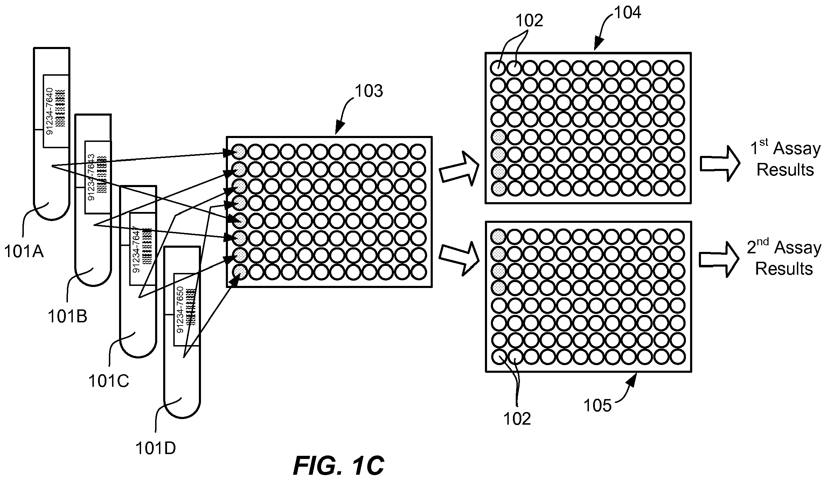

As shown in FIG. 1A, a typical polymerase chain reaction (PCR) micro-well plate 100 used in PCR testing contains a plurality of micro-wells 102 that provide test positions (e.g., 8 x 12 = 96 potential test positions). Following sample preparation on a deep well extraction plate, the PCR micro-well plate 100 receives eluted samples from the extraction plate as well as mastermix and possibly other components specifc to the assay being conducted and undergoes processing in an AD device to replicate and quantify the component of interest (e.g., nucleic acid). In such PCR testing, the per test, per patient cost of PCR assay materials is increased when any potential test postions (e.g., test wells) in the PCR plate are unused when an assay is run. Additionally, the per test, per patient cost may be increased when manual preparation is used in place of automated preparation due to high labor cost components.

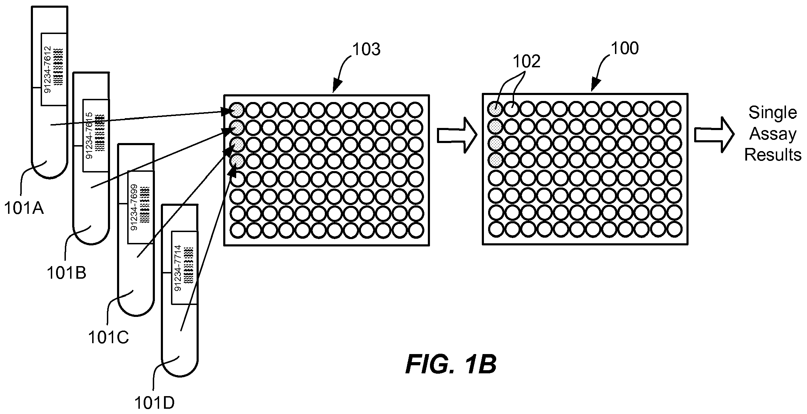

As shown in FIG. 1B, kPCR sample preparation ("SP") on a PCR test plate 100 has historically been either prepared manually, or automatically prepared by using a fixed assay selection and a fixed plate layout on the PCR micro-well plate 100. Patient samples from multiple sample containers (e.g., 101A-101D) are transferred to deep wells of an extraction plate 103. These patient samples are then processed via sequential additions of reagent, magnetic particles, wash buffers, elution buffer, and possibly other components to produce patient sample eluates (purified samples). These patient sample eluates are then transferred to the PCR micro-well plate 100 and each undergoes the same assay on the PCR micro-well plate 100. Manual sample preparation is very tedious for the preparer, and due to the very large number of maunal steps required to achieve a complete sample preparation while coordinating addition of patient samples, reagents, magnetic particles, wash buffers, elution buffer, and assay mastermix, may be subject to a high occurrence of human preparation error.

Automated sample preparation, although it may reduce the human preparation error, has historically been inflexible with fixed assay selection and a fixed plate layout. In one prior art system as shown in FIG. 1B, only one assay is conduced on the PCR micro-well plate 100 thus providing a single result per patient sample for a single assay only. To provide further flexibility, split plate assays have been used wherein patient sample is transferred to multiple extraction plate wells of the extraction plate 103, and then processed eluate from those multiples of patient samples are transferred to first and second test plates 104, 105 wherein a first assay is conducted on the first test plate 104, and a second assay is conducted on the second test plate 105. Thus, first assay results are produced for the patient samples from the first test plate 104, and second assay results are produced for the patient samples from the second test plate 105. Although these prior art methods operate as intended, they tend to be error-prone (manual) or are generally inflexible, i.e., one assay per test plate.

Therefore, methods and apparatus that improve efficiency and cost effectiveness of automated sample processing (e.g., PCR processing) are desired. An example of an automated nucleic acid analysis method is described in US2013/0157273.

### SUMMARY

According to a first aspect, a method of preparing samples for assaying on a test plate having a plurality of wells is provided. The method includes receiving, via a user interface, instructions to run a first assay type and a second assay type, determining sample identifications for a plurality of samples to be assayed, receiving assay orders corresponding to the sample identifications, defining, based on the assay orders and sample identifications, first and second assay regions of the plurality of wells, wherein each defined assay region includes at least one of the plurality of wells, and creating an electronic plate map including a graphical representation of the test plate indicating the first assay region and the second assay region and instructions to run a first assay type on samples contained within the first assay region, and to run a second assay type on samples contained within the second assay region, wherein the electronic plate map includes and defines two or more assay regions, which are each a collection of physical well locations on the test plate.

In another aspect, the method includes performing sample processing of multiple patient samples in multiple extraction plate wells of an extraction plate to prepare multiple eluted samples in at least some of the multiple extraction plate wells, and transferring, based upon the electronic plate map including multiple assay regions, one of the multiple eluted samples from a single extraction plate well of the multiple extraction plate wells into multiple test wells of the test plate, wherein each of the multiple test wells of the test plate are in a different one of the multiple assay regions and each is configured to carry out a different assay type.

According to another aspect, a method as described above is provided, wherein the test plate is a PCR plate having a plurality of PCR plate wells. The method includes providing a plurality of patient samples to be assayed, the patient samples including sample identifications, receiving assay orders corresponding to the plurality of patient samples from an LIS system, selecting, based upon the assay orders and sample identifications, a number and type of assays to perform on the PCR plate, dynamically defining assay data sets for each of the types of assays including types and numbers of pre-processed controls and post-processed controls, preparing the plurality of patient samples and the pre-processed controls through to patient sample eluate and control sample eluate on extraction plate wells of an extraction plate, defining, based on the types of assays, number of assays to perform, and a number of controls, an electronic plate map including multiple assay regions including a plurality of PCR plate wells, wherein each of the multiple assay regions includes at least one of the plurality of PCR plate wells, and dispensing patient sample eluate from an extraction plate well into the multiple assay regions wherein the number of multiple assay regions on the PCR plate is 2 or more; and a different assay is to be performed in each of the multiple assay regions.

According to another aspect, a sample preparation apparatus is provided. The sample preparation apparatus includes an extraction plate, a test plate, a memory operative to store at least patient sample identifications, assay orders for each patient sample, and number and type of assays, a processor coupled to the memory and operable to generate an electronic plate map including a graphical representation of the test plate indicating the multiple assay regions based at least on a number of the patient samples, a number and type of assay orders for each of the patient samples, and number of controls, and one or more robots each including one or more coupled pipettes configured to patient sample eluate from an extraction plate well of the extraction plate and master mix to the multiple assay regions.

According to another aspect, a machine-readable medium having non-transient instructions stored thereon is provided. The machine-readable medium includes a dynamic run setup module configured to define a plurality of assay data sets with desired run configurations based at least in part on a number and type of assays to run, and number and type of controls, and a work list and plate map display module configured to generate an electronic plate map including a graphical representation indicating multiple assay regions of a test plate of patient sample eluate and control eluate and optionally one or more post-processed controls.

Still other aspects, features, and advantages of the present invention may be readily apparent from the following detailed description by illustrating a number of example embodiments and implementations, including the best mode contemplated for carrying out the present invention. The present invention may also be capable of other and different embodiments, and its several details may be modified in various respects, all without departing from the scope of the present invention. Accordingly, the drawings and descriptions are to be regarded as illustrative in nature, and not as restrictive. The drawings are not necessarily drawn to scale. The invention is to cover all modifications, equivalents, and alternatives falling within the scope of the invention.

### BRIEF DESCRIPTION OF THE DRAWINGS

The drawings, described below, are for illustrative purposes only and are not necessarily drawn to scale. The drawings are not intended to limit the scope of this disclosure in any way.
FIG. 1A illustrates a perspective view of a PCR test plate according to embodiments.
FIG. 1B illustrates a sample preparation apparatus and method according to the prior art.
FIG. 1C illustrates another sample preparation apparatus and method according to the prior art.
FIG. 2 illustrates a sample preparation apparatus configured to carry out a sample preparation method according to embodiments.
FIG. 3 illustrates transfer of patient samples to extraction plate wells of an extraction plate and the subsequent transfer of eluted samples to test plate wells of a test plate (e.g., PCR plate) including multiple assay regions according to embodiments.
FIG. 4 illustrates a flowchart of a sample preparation method of according to embodiments.
FIG. 5A illustrates a flowchart of another sample preparation method of according to embodiments.
FIG. 5B illustrates a flowchart of another sample preparation method of according to embodiments.
FIG. 6 illustrates various modules of a sample preparation software program adapted to carry out the sample preparation method according to embodiments.
FIG. 7 illustrates a work flow module screen of a workflow start module a sample preparation software program adapted to carry out a portion of the sample preparation method according to embodiments.
FIG. 8 illustrates a dynamic assay run setup screen of a dynamic assay run setup module of a sample preparation software program adapted to carry out a portion of the sample preparation method according to embodiments.
FIG. 9 illustrates a work list and plate map display screen of a work list and plate map display module of a sample preparation software program adapted to carry out a portion of the sample preparation method according to embodiments.
FIG. 10 illustrates a required inventory screen of a required inventory module of a sample preparation software program adapted to carry out a portion of the sample preparation method according to embodiments.
FIG. 11 illustrates a SP reagent carrier loading screen of a required inventory module of a sample preparation software program adapted to carry out a portion of the sample preparation method according to embodiments.
FIG. 12 illustrates a patient sample usage summary screen of a patient sample usage module of a sample preparation software program adapted to carry out a portion of the sample preparation method according to embodiments.
FIG. 13 illustrates a sample preparation reagent carrier loading screen of a required inventory module of a sample preparation software program adapted to carry out a portion of the sample preparation method according to embodiments.
FIG. 14 illustrates a status summary screen of a status module of a sample preparation software program adapted to carry out a portion of the sample preparation method according to embodiments.
FIG. 15 illustrates a sample preparation run report screen of an error report module of a sample preparation software program adapted to carry out a portion of the sample preparation method according to embodiments.

### DESCRIPTION

Reference will now be made in detail to the various embodiments of this disclosure, examples of which are illustrated in the accompanying drawings. Wherever possible, the same reference numbers will be used throughout the drawings to refer to the same or like parts. The definitions section below provides definitions of some of the terminology used herein.

As discussed above, due to the fixed format of preparation on the test plate, many of the potential tests positions (e.g., test plate micro-wells) in a PCR plate may remain unused in existing automated processing methods. Alternatively, a low sample preparation throughput within the lab may result from allowing for an adequate number of assay orders to be placed and to more fully populate the PCR plate, but at the expense of high labor cost, increased risk of errors, and variability.

Accordingly, one or more embodiments of the present invention provide sample preparation methods and sample preparation apparatus including improved flexiblity, throughput, and/or efficiency.

In a first broad aspect, sample preparation method and sample preparation apparatus embodiments of the present invention provide the ability to run a large number of different patient samples, and a large number of assay types (e.g., two or more assay types per patient sample) on a single test plate (e.g., PCR plate). In some embodiments, three or more assay types, four or more assay types, five or more assay types, or even six or more assay types may be run per patient sample. The multiple assay types may be included in multiple assay regions provided on the test plate (e.g., PCR plate), each of which may receive different master mixes. Thus, the sample preparation apparatus and methods provide extreme flexibility in terms of selection of patient samples, assays per patient sample, assay kits, and the types and numbers of controls desired per assay.

The apparatus and methods produce, as a deliverable, an electronic plate map that may be displayed to the user, printed, and may later be used by an amplification and detection device (AD device) to aid in processing the various sample assays. The assay results may be returned to the sample preparation apparatus, which may interface assay results with a Laboratory Information System (LIS). In another embodiment, the sample preparation apparatus and method may allow the use of pre-processed controls, post-processed controls, or both. Other controls, such as universal negative controls may be included as part of the sample preparation process.

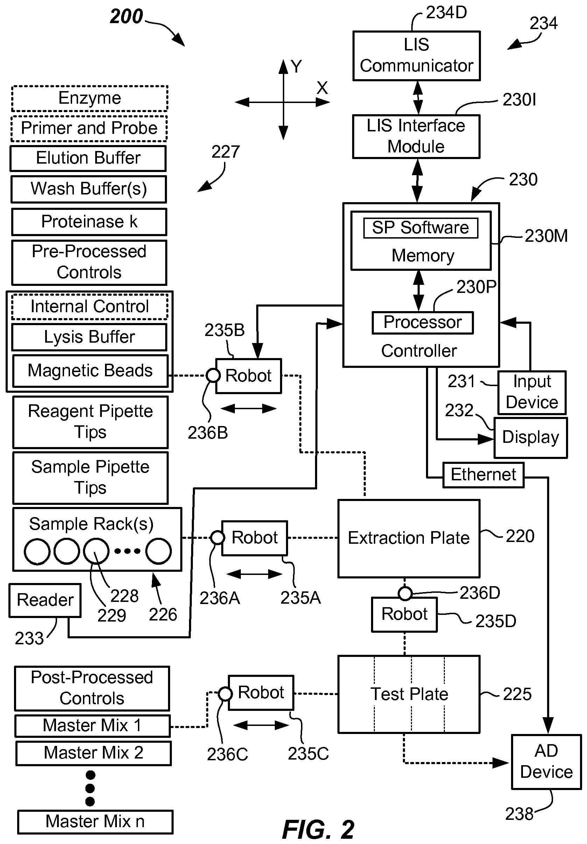

These and other aspects and features of embodiments of the invention will be described with reference to FIGs. 2-15 herein.

FIG. 2 through FIG. 15 will be referred to herein to fully explain the sample preparation method (hereinafter "SP method") and the sample preparation apparatus (hereinafter SP "apparatus 200") that are provided according to embodiments.

In a first aspect, as best shown in FIG. 2, the SP apparatus 200 includes an extraction plate 220, which may be a 96 well (e.g., 8x12), deep-well plate, and a test plate 225, which may be a PCR test plate. Test plate 225 may include 96 (e.g., 8x12) wells. However, it should be apparent that the extraction plate 220 and the test plate 225 may have other configurations (e.g., different numbers of wells, or different numbers of rows and columns). Any suitable configuration may be used. The SP apparatus 200 may further include one or more sample racks 226 that may be configured to contain patient samples 228 that may be contained in sample containers 229 (e.g., sample tubes). Patient samples 228 may be urine, whole blood, blood serum or plasma, swab extracts from urogenital, nasopharyngeal, buccal or eye swabs, cerebral spinal fluid, semen, stool, breast milk, saliva, sputum, cell culture, amniotic fluid, ascites, bronchial alveoli lavage (BAL), collection media, peripheral blood mononuclear cells (PBMC), buffy coat, or the like. The sample racks 226 may be loaded onto one or more autoload trays, and may be automatically loaded via a prompt in accordance with some embodiments of the method. Upon being loaded into the SP apparatus 200, a reader 233 may read a sample rack identifier, sample container identifiers on each sample container, and sample slot locations (i.e., spaced locations in the sample rack 226). This sample identification data on patient samples and their location in the sample racks 226 may be stored in memory 230M of a controller 230 of the SP apparatus 200.

The SP apparatus 200 may include one or more access areas 227 that are configured to contain consumables. The consumables may include components that are used in portions of the SP method 400 that may be taking place on the extraction plate 220 or later on the test plate 225. Consumables may include plates, pipette tips, one or more Reagents, wash buffers, elution buffer, lysis buffer, magnetic beads, pre-processed controls, post-processed controls, internal controls, Proteinase K, and/or other processing components. Consumables may be loaded onto one or more autoload trays, which may be automatically loaded into the SP apparatus 200, such that they reside at, and are accessible from, the one or more access areas 227 in accordance with one or more aspects.

The SP apparatus 200 may include the controller 230 including a processor 230P and the memory 230M. Processor 230P may include any suitable microprocessor or other processing device adapted to execute sample preparation (SP) software program instructions and interface with memory 230M and various other components of the SP apparatus. Processor 230P may be a windows-based computer, for example. Memory 230M may be operative to store at least patient sample identifications of patient samples 228 from the sample containers 229, the location of each in the sample rack 226, assay orders for each patient sample 228, and number of different assays types to be performed. Processor 230P may be coupled to the memory 230M and may execute the SP software program to generate an electronic plate map, as will be apparent from the following. The electronic plate map includes information about the multiple assay regions based at least on the number of patient samples and number and type of assay orders for each patient sample, and number of controls for each assay type.

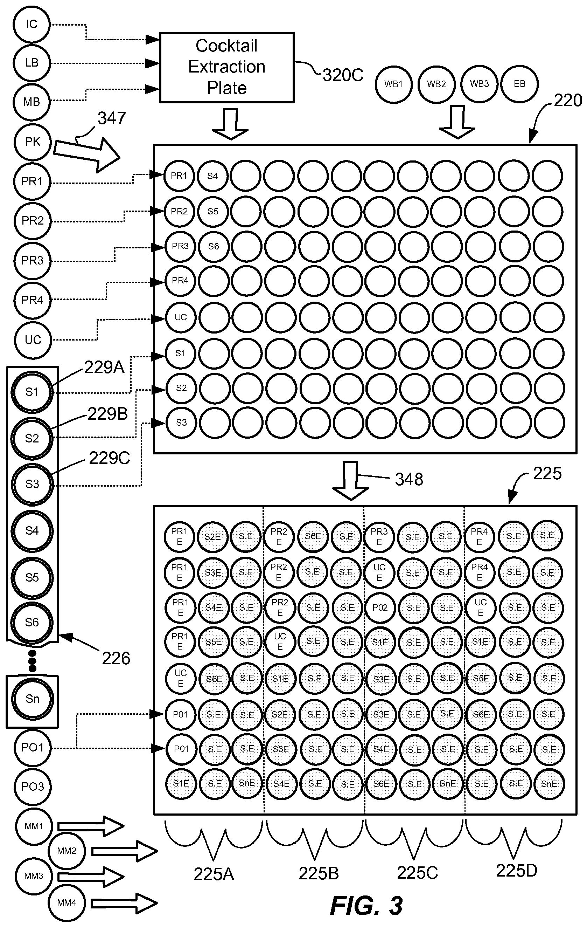

A plurality of robots (e.g., robots 235A, 235B, 235C, 235D) having one or more pipettes (e.g., pipettes 236A, 236B, 236C, 236D) coupled to and moveable thereby may be controlled by drive signals from the controller 230 to cause various movement of the pipettes 236A, 236B, 236C, 236D in coordinate space (e.g., X and Z and/or X, Y, and Z, wherein Z is into and out of the paper in FIG. 2). One or more robots (like robot 235A), for example, may be used to move one or more pipettes (like pipette 236A) in two or more coordinate directions in order to aspirate patient samples 228 from the sample containers 229 residing in the sample rack 226, and dispense a predefined volume of the patient samples 228 into extraction wells in the extraction plate 220, as shown in FIG. 3.

For example, patient sample S1 may be aspirated from a first sample container 229A received in a first slot of the sample rack 226 and then dispensed to an extraction plate well of the extraction plate 220, patient sample 2 229B (labeled S2) may be aspirated from sample container 229B and dispensed to another extraction plate well of the extraction plate 220, patient sample S3 may be aspirated from sample container 229C and dispensed to another extraction plate well, and so on. Depending upon the number of different assay types to be run, the extraction wells may include multiple patient samples (e.g., S1 to S6), calibrators, other controls (e.g., universal controls UC) such as negative controls and/or pre-processed controls (e.g., pre-processed controls PR1-PR4). Any suitable type of control may be used. The controls may include calibrators, pre-processed controls, or any other type of pre-extracted material, and combinations thereof, for example.

Likewise, one or more robots (like robot 235B) may be used to aspirate and dispense some of the consumables. Consumables may include a lysis buffer, magnetic beads, and internal controls (if used). Consumables may include reagents such as Proteinase K, wash buffers, and/or elution buffers. In some embodiments, the lysis buffer, magnetic beads and internal controls (if used) may be premixed to form a lysis cocktail that may be contained in wells of cocktail extraction plate 320C, such as a 96-well deep well plate. Throughout the SP method 400, the remaining liquid levels of the various consumables (e.g., wash buffers, elution buffer, Proteinase K, magnetic beads, lysis buffer, and internal controls - if used) may be monitored and stored. Likewise, remaining levels of patient samples S1-Sn and/or pre-processed controls (PR1-PR4) may be monitored and stored. Other consumable items may be monitored, as well.

Any suitable aspiration and dispensing system may be used for the aspiration and dispensing of patient samples 228 and various consumables, such as described in US 5,777,221; US 6,060,320; US 6,158,269; US 6,250,130; US 6,463,969: US 7,998,751; US 7,205,158. Other suitable aspiration and dispensing systems may be used. Pipette tips may be replaced after each dispense from a supply of reagent pipette tips and sample pipette tips.

In order to carry out the SP method 400, controller 230 may receive Tech input from an input device 231. Input device 231 may be a keyboard, mouse, touchscreen, or combinations thereof. Further, a display 232 may display various screens of software modules of a sample preparation software program (SP software program) used to execute the sample preparation method 400. Display 232 may be any suitable display device, such as a screen or other display device, for example. The display 232 may display various screens generated by the SP software program including a listing of selectable dynamic protocols, a dynamic assays setup screen, a work list correlating the patient samples 228 with assay orders, lists or images of pre-extract controls and post-extract controls to be used, load time reminders for post-extract controls, partial or full lists of required inventories, and possibly their placement location on the one or more carriers or auto load trays, a sample container usage summary, a patient sample usage summary, a status summary, a sample preparation report, and/or graphical representations of plate maps.

As discussed above, controller 230 may also receive input from a reader 233, which may be a barcode reader, radio frequency identification device (RFID), or other suitable reader device configured to read an identifier (e.g., device, tag, barcode, or other readable indicia) on each of the sample containers 229. Reader 233 may also read identifiers on the sample racks 226, and/or the slot position of the sample containers 229 within the sample rack 226. Thus, the controller 230 may include an internal database stored in the memory 230M that correlates the identification of the patient samples 228 (e.g., S1 to Sn) with sample rack 226 and rack slot position in the sample rack 226 containing them.

Controller 230 may also receive input from a laboratory information system 234 (hereinafter LIS 234). LIS 234 may include a LIS communicator 234D. LIS communicator 234D may interface and communicate digitally with controller 230 through an LIS interface module 2301, which may receive assay order information from the LIS communicator 234D for patient samples 228, and also return result files and/or other information to the LIS communicator 234D. Communication between the LIS interface module 2301 and the LIS communicator 234D may be by using ASTM 1394 and 1381 protocols for example. Other suitable communication protocols may be used.

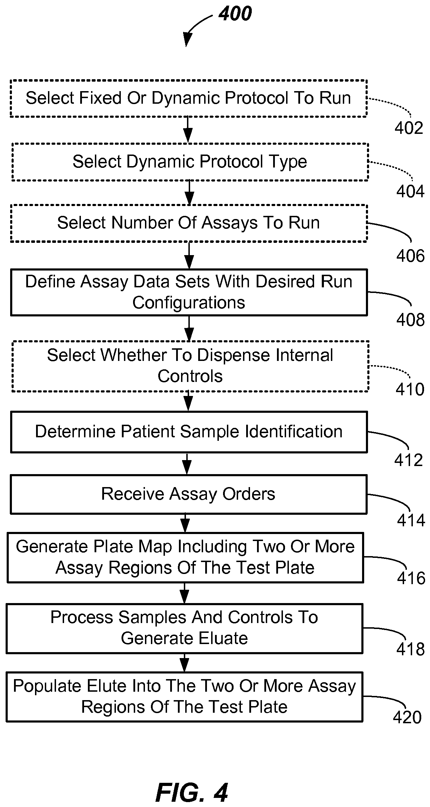

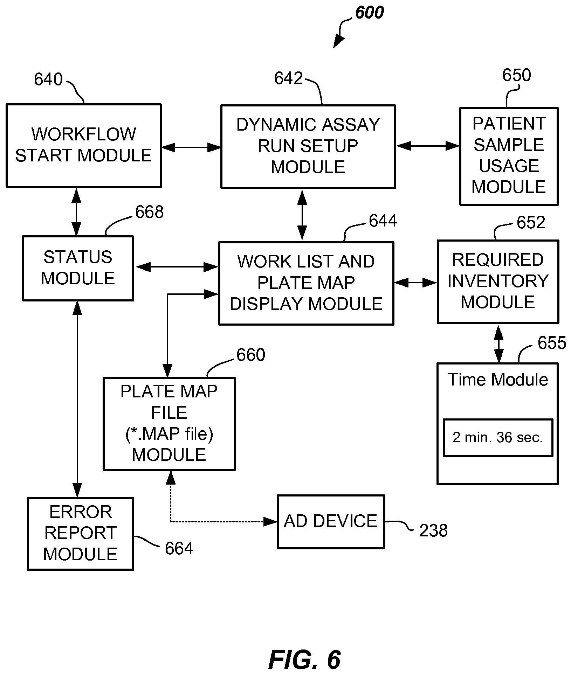

The sample preparation (SP) method 400 and SP software program 600 and various modules thereof will now be explained in more detail, with reference to FIGs. 4 through 15. The SP method 400 may start at a workflow start module 640 (FIG. 6) of the SP software program of the SP method 400 by allowing the Tech to optionally select whether the desired assays to be run are dynamic or fixed. "Dynamic assay" as used herein means that more than one assay type will be run on the test plate 225 for at least some of the patient samples (S1-Sn) that are contained in an extraction plate well of the extraction plate, and that the combination of assay types and controls may be dynamically selectable. "Fixed assay," as used herein means that one and only one assay will be run for each eluted sample contained in each extraction plate well, and that only one assay type per test plate is run.

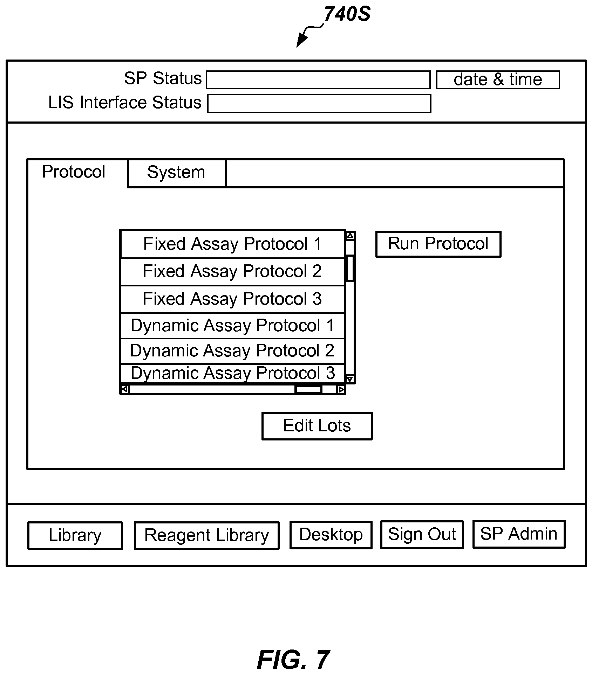

An example work flow screen 740S of the workflow start module 640 is shown in FIG. 7. Within the workflow start module 640, the Tech can select between fixed or dynamic assay protocols (see also block 402 of FIG. 4). In the depicted embodiment, more than one fixed assay protocol may be selected, such as Fixed Assay Protocol 1 through Fixed Assay Protocol 3. Such fixed assay protocols may be any assay protocol that currently exists, and thus the sample preparation for the fixed assay protocols may be carried out in accordance with prior art methods.

In accordance with embodiments of the invention, at the workflow start module 640, the Tech may select to run a dynamic assay protocol (see also block 402 - FIG. 4). In the depicted embodiment, more than one dynamic assay protocol type may be selected (see block 404). For example, dynamic assay protocol 1 through dynamic assay protocol 3 are shown, and one dynamic assay protocol may be selected. More or less numbers of dynamic assay protocol types may be presented as options. Dynamic assay protocol 1 through dynamic assay protocol 3 may vary from each other in terms of extraction process steps, the volume of sample and/or buffer aspirated, incubation time and temperature, and/or PCR reaction components dispensed, or other assay specific factors.

Once the Tech selects a dynamic assay protocol type (e.g., dynamic assay protocol 1 through dynamic assay protocol 3), they may select RunProtocol and then commence to the selection portion of the SP method 400 where various data sets may be defined. In FIG. 4, the selection of a dynamic assay protocol or fixed assay protocol in block 402 may be optional. For example, in some methods, the SP apparatus 200 may be dedicated to running only dynamic protocols, thus no selection may be required. Likewise, selection of the dynamic assay protocol type in block 404 may be optional, as some methods and some SP apparatus may be simplified to running only one particular type of dynamic assay protocol.

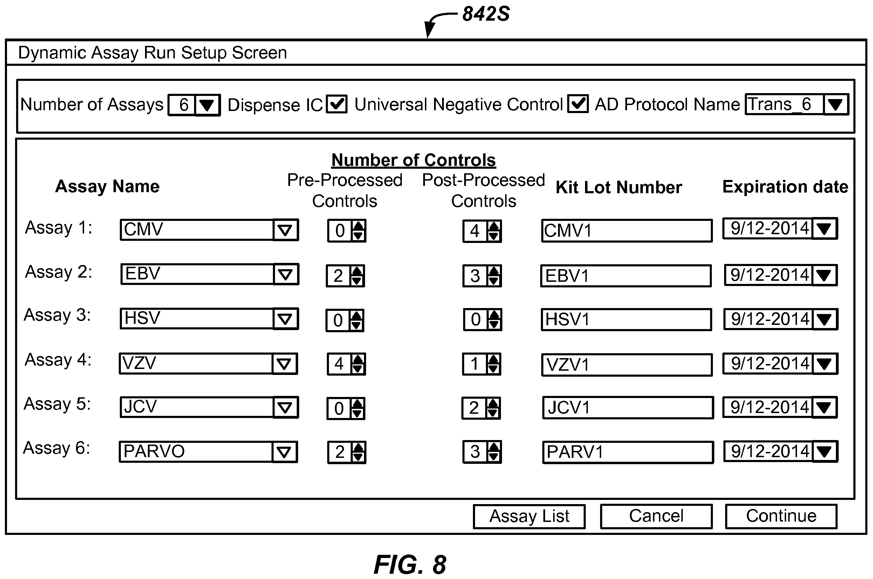

The SP method 400 and SP Apparatus 200 may also include in the SP software program, a dynamic assay run setup module 642 as shown in FIG. 8. Within the dynamic assay run setup module 642, the Tech may, in block 406 (FIG. 4), select a number of assay configurations to run. Thus, the SP program may receive as an input, via a user interface (including the input device 231 and display 232), instructions to run a first assay type and a second assay type. The number of assays to run may be two or more, three or more, four or more, five or more, or even six or more, for example. This block 406 is also shown as optional, as in some embodiments of the SP apparatus, a fixed number of dynamic assay types may be predefined. However, for maximum flexibility for small-scale labs, dynamically defining the assay types to be used provides excellent flexibility to accommodate varying test workloads.

Once the number of assays to run is selected, the Tech may define assay data sets with desired run configurations in block 408. The defined assay data sets may include various combinations that may be defined from selectable options on a dynamic assay run setup screen 842S that may be displayed on display 232, as shown in FIG. 8. Each defined assay data set (e.g., six data sets shown) may include at least the assay name (e.g., CMV, EBV, HSV, VZV, JCV, PARVO are shown), number of pre-processed controls, and number of post-processed controls. The assay data set may also include kit lot number, expiration date, for example. Thus, it should be understood that multiple data sets may be selected with various combinations of pre-processed controls and post-processed controls. In some embodiments, one or more of the assay data set may be selected and populated off from a separate list of possible assay run configurations. In some embodiments, the use of an internal control (IC) may be optionally selected in block 410. In the embodiment shown, the use of IC is provided as a global variable and is applied to all assays, if selected. Optionally, the use of internal controls (ICs) may be provided as a per-assay selectable variable, much like pre-processed controls.

In accordance with another aspect, the SP method 400, in 412, determines patient sample information. SP software program may initiate a drive signal to a motor and thus pull/load the sample carrier (e.g., containing one or more sample racks 226) onto a deck of the SP apparatus 200. SP software program may read all sample rack IDs (e.g., IDs on sample rack 226), patient sample ID's (e.g., barcodes on each sample container 229), and sample slot locations (e.g., barcodes or other readable indicia at each slot location) for all sample containers 229 (e.g., sample tubes) contained on the sample rack 226. The reading may be performed by any suitable reader 233, such as barcode or other reader device described herein. Thus, information about sample identification, identification of the sample rack 226, and the exact slot location on the sample rack 226 for each sample container 229 is determined. Thus, the SP software program may determine patient sample identifications for a plurality of patient samples to be assayed in block 412.

In block 414, the SP method 400 may receive assay orders. The LIS 234 (if available, connected, communicating) may be queried and respond with the assay orders associated with open orders in the LIS 234 for each of the sample IDs for each patient sample 228 that have been read by reader 233 and stored in memory 230M. The SP Software may perform a first-pass/recommended population of the work list file of the work list and plate map display module 644 based on the previously read sample IDs and open assay orders from the LIS 234, and a dynamic assay process/run configuration (based on the number of patient samples, number of assay types, types of assays per patient sample, and number of controls per assay type). The SP software program may tally the number of patient samples and controls per assay type and define the dynamic TDef configuration, which includes at least a first region and a second region of the test plate 225. Dynamic assay run configuration is defined herein as the user defined layout and population of the PCR plate. The dynamic run configuration may be populated in any order.

In one or more embodiments, a recommended population of the work list 945 may be modified by the Tech. The Tech may review the patient samples 228, and the recommended assay orders, and make changes as they see fit by changing the selections on the work list 945 of the work list and plate map display screen 944S. Any patient sample 228 that does not have an order in the LIS 234 may be flagged (e.g., by being displayed as a separate color) be manually entered by the Tech in the work list 945, such as STAT samples. Calibrator samples may also be entered if provided in the sample rack 226, and treated like any other patient sample.

Also, if the LIS 234 is not communicating, the assay orders may be received by being manually populated by the Tech by entering a sample ID and toggling the desired assay selections on the work list 945. When the LIS 234 is again connected, any new assay orders that have been manually entered may be transmitted to the LIS 234 by LIS interface module 2301.

In block 416, the SP method 400 utilizing the plate map file module 660 of the SP software program may generate (e.g., create) an electronic plate map. The plate map may be generated (e.g., created) within the work list and plate map display module 644. The electronic plate map may be a data file that contains a compilation of sample information, assay type, controls information, corresponding test plate well location and assay region information based on the dynamic TDef. Thus, block 416 involves defining, based on the received assay orders from block 412 and sample identifications from 412, at least first and second assay regions of the plurality of test plate wells of the test plate 225, wherein each defined assay region includes at least one of the plurality of test plate wells. Thus, electronic plate map includes and defines two or more assay regions, which are each a collection of physical well locations on the test plate 225. Depending upon the capability of the SP apparatus 200, multiple assay regions may be provided, such as two to six, or more.

The electronic plate map includes instructions generated by the plate map file module 660 to run a first assay type on samples contained within the first assay region, and to run a second assay type on samples contained within the second assay region. If more than two assays are selected in block 406, then further instructions are provided to run additional assay types on sample eluate and possibly control eluate or controls contained within the additional assay regions.

The electronic plate map may be based at least upon the number and identity of the patient samples, and the number and type of assays to be performed for each patient sample. The electronic plate map may also be based on, number of post-processing controls, number of pre-processing controls, number of other controls (e.g., universal controls and/or calibrators), and possibly other information. The number and location/size of the multiple assay regions of the test plate 225 may be set by the SP software program. Each assay region may be selected as a zone on the PCR plate. Assay regions may be provided in any suitable pattern on the test plate 225. Each may be of a different size, and may be of different shapes. Assay regions may be any suitable polygonal shape.

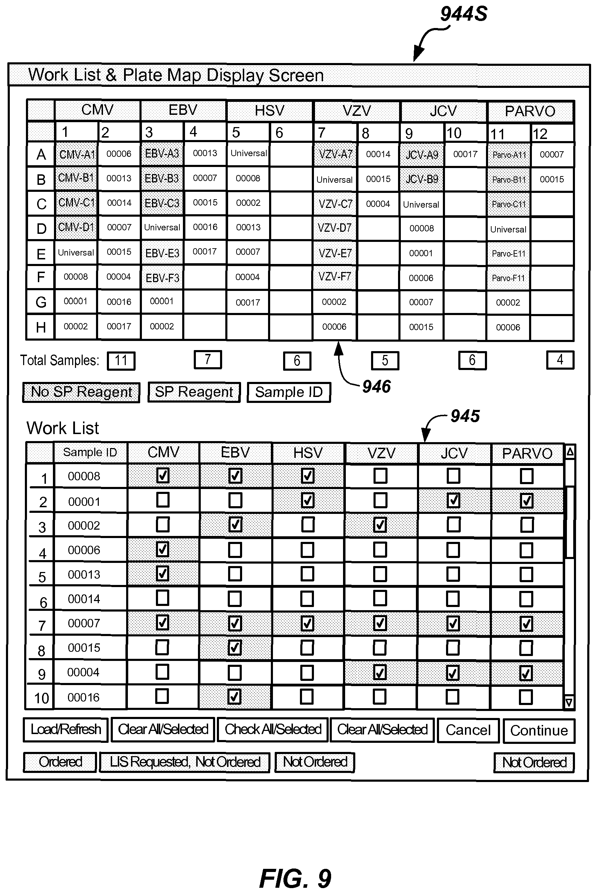

The contents of the electronic plate map may be displayed as a graphical representation on the display 232 as a plate map 946, as shown in FIG. 9. Thus, in one or more embodiments, the SP method 400 displays a graphical representation of the test plate 225 indicating at least a first assay region and a second assay region. Other assay regions may be included, depending on the number of assay types selected. Six assay regions (CMV, EBV, HSV, VZV, JCV, PARVO) are shown as equal-sized columns in FIG. 9, but other numbers of zones and sizes of zones may be selected. Color coding may be provided for visual effect. For example, pre-processed control eluate may be indicated with one color, while patient sample eluate, post-processed controls and other controls may be designated by other colors.

In some embodiments, the plate map 946 may be displayed as a part of the work list and plate map display screen 944S. In the depicted embodiment, the plate map 946 and the work list 945 are displayed on the same work list and map display screen 944S. However, they could be displayed on separate display screens in some embodiments. A Tech may make manual changes (edits) to the work list 945 and those changes may immediately be graphically illustrated on the plate map 946. Accordingly, the Tech and SP software program can help plan to ensure complete or nearly complete utilization and population of the test wells of the test plate 225 before running the sample preparation on the extraction plate 220.

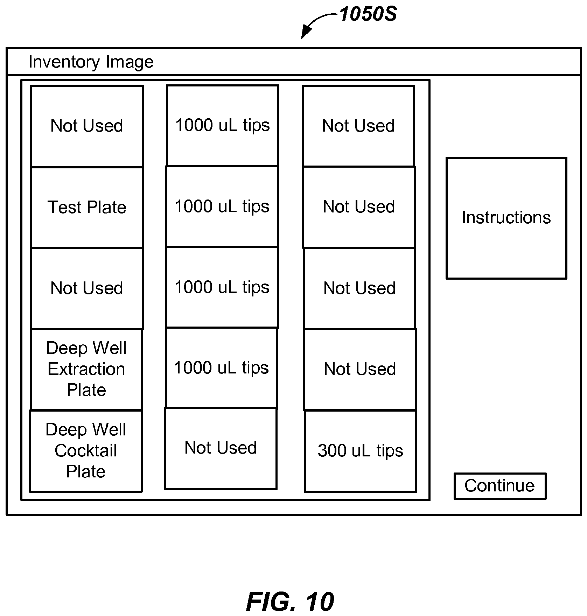

In some embodiments, the SP software program may include a required inventory module 652 as shown in FIG. 6. Required inventory module 652 may prepare a list or visual image of some or all of the inventory requirements that may be needed to run the various assays in accordance with the SP method 400. The inventory requirements may be based upon information in the electronic plate map file, for example. Thus, inventory requirements may include, in one embodiment, deep well extraction plates, PCR test plate, and numbers of pipette tips (of one or more sizes). A list or image file maybe created and may be displayed as an inventory list or image on the display 232 or may be provided in other suitable format (e.g., a hard copy printout). In some embodiments, a graphical inventory image 1050S, as shown FIG. 10, may graphically instruct the Tech where and how many of an inventory item to load onto a given location on a carrier or tray (e.g., an autoload tray) of the SP apparatus 200. Instructions may be provided along with the graphical inventory image 1050S. When the Tech has loaded the autoload tray, then they may select Continue and the tray may be loaded into the SP apparatus 200.

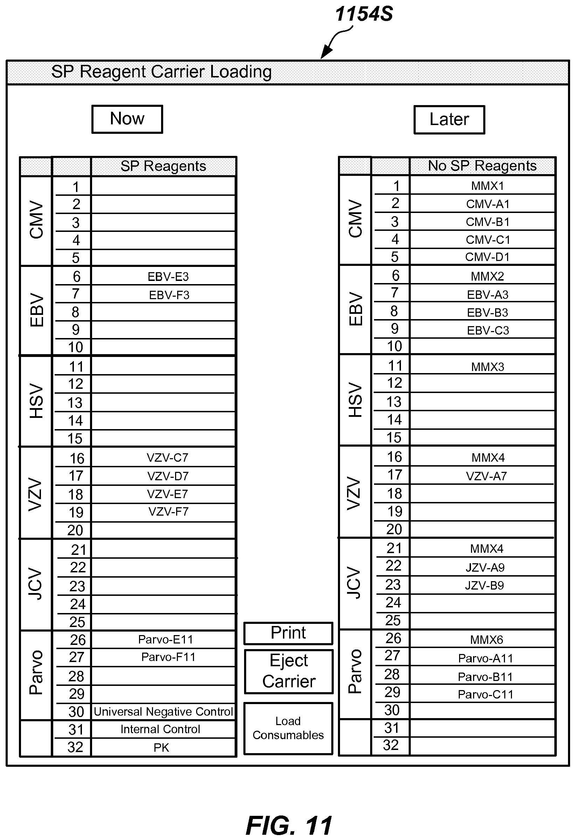

Similar inventory images may be provided by the required inventory module 652 for other inventory items, such as for sample preparation SP consumables and No SP consumables (e.g., pre-processed controls, post-processed controls, universal controls, proteinase K, internal controls, lysis buffers, magnetic particles, wash buffers, elution buffers, master mixes, enzyme mix, primer and probe mix, or the like). SP consumables are consumables used on the extraction plate 220, whereas No SP consumables are used later in processing taking place on the test plate 225. Inventory
images may instruct the Tech where and/or how many of each of the consumables to load on a given carrier or tray (e.g., an autoload tray). Inventory images may be provided to aid the Tech in loading consumable items on multiple trays of the SP apparatus 200. In this manner, consumables waste may be minimized, and disruptions and/or off-line time may be avoided. FIG. 11 illustrates an SP analyte reagent carrier loading screen 1154s. This screen displays an image and/or otherwise provides (e.g., a printed list) a list of the various SP reagents and No SP reagents for each selected assay type and their location on a SP carrier and a No SP carrier.

In block 418, the SP method 400 may then process the patient samples, as well as the selected controls, on the extraction plate 220 to produce eluted samples (eluate) at the end of the processing phase of the extraction plate 220. The eluted samples may include eluted patient samples and eluted control samples.

With reference to FIGs. 2 and 3A, an example method for the processing of the patient samples and controls on the extraction plate 220 will be further described. As a first step in the extraction plate processing sequence, a reagent such as Proteinase K (PK) may be dispensed by the robot 235B of the SP apparatus 200 to dispense PK to all extraction plate wells of the extraction plate 220 that will receive a patient sample 228 or a control (i.e., all that will be populated) based upon the work list 945 and the electronic plate map. The arrow 347 designates that all extraction plate wells that will be populated may receive PK.

The SP apparatus 200 may also dispense controls and/or calibrators. As depicted, an ordered pre-processed control (e.g., PR1 - a pre-processed control for a first assay type 1 being selected) may be aspirated and dispensed by the one or more robots 235B and coupled pipettes 236B to an extraction plate well of the extraction plate 220, as shown. Likewise, a universal control (UC), if selected, may be aspirated and dispensed by the one or more robots 235B and one or more coupled pipettes 236B to one or more extraction plate wells of the extraction plate 220, as shown. Additional ordered pre-processed controls (e.g., PR2-PR4 - pre-processed controls for assay types 2-4 that have been selected) may be aspirated and dispensed by the one or more robots 235B and one or more coupled pipettes 236B to extraction plate wells of the extraction plate 220, as shown. As should be apparent, zero, one, or more than one pre-processed controls may be selected by the Tech when running the dynamic assay run setup module 642. Internal controls and/or universal controls (UC) may also be
dispensed. A clean pipette may be picked up by the one or more robots 235B for each new aspiration and dispense of a different pre-processed control or other control.

Based on the number of assays selected by the Tech in the dynamic assay run setup module 642, a number of assay regions on the test plate 225 (e.g., PCR test plate) are designated. The sizes or each of the assay regions are selected based on the number of patient samples having that particular assay being ordered, the number or pre-processed controls, the number of post-processed controls for that assay, and the number of other controls or calibrators. In the depicted test plate 225 (e.g., PCR test plate) of FIG. 3, four assay regions 225A, 225B, 225C, and 225D are provided, based upon four assays being selected in the dynamic assay run setup module 642. However, other numbers of assay regions may be selected, such as two or more, three or more, four or more, five or more, or even six or more plate regions. Each assay region may be dedicated to a certain assay, such as CMV, EBV, JCV, BKV, HSV, VZV, HHV-6, Parvo, or Adeno, for example. Other types and numbers of assays may be run.

Any combination of selected pre-processed controls (e.g., PR1, PR2, PR3, PR4) for those assay types may be provided to extraction plate wells of the extraction plate 220. Likewise, patient samples (e.g., S1 - Sn shown) may be aspirated and dispensed by the one or more robots 235A and coupled pipettes 236A directly to one or more extraction plate wells of the extraction plate 220. Depending upon the numbers of controls and number of assays selected in the in the dynamic assay run setup module 642, between about 1 and about 96 patient samples may be dispensed to the extraction plate 220, for example. Other numbers of patient samples may be dispensed based upon the size of the test plate 225 that is used.

Likewise, internal controls (if selected), lysis buffer and magnetic beads may be aspirated and dispensed by one or more robots 235B and one or more coupled pipettes 236B directly to each of the populated extraction plate wells of the extraction plate 220 containing patient samples S1-Sn or controls (e.g., PR1, PR2, PR3, PR4, and/or UC). In some embodiments, the internal controls (IC), if selected, lysis buffer (LB), and magnetic beads (MB) may be provided as a premixed cocktail on a cocktail extraction plate 320C as shown in FIG. 3. Once the Proteinase K, pre-processed controls (e.g., PR1-PR4), other controls (e.g., universal controls (UC) and/or calibrators), patient samples (S1-Sn), have been dispensed onto the extraction plate 220, the premixed cocktail of internal controls (IC) if selected, lysis buffer (LB), and magnetic beads (MB) may be dispensed to each occupied extraction plate well of the extraction plate 220. The large arrow indicates that all populated extraction plate wells receive the premixed cocktail from cocktail extraction plate 320C.

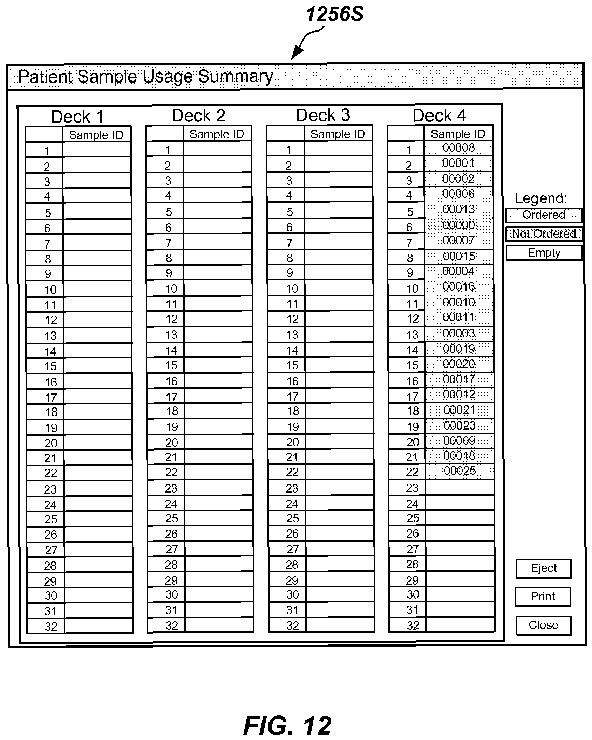

As aspiration and dispensing takes place, various used and/or remaining volumes of the patient samples, proteinase k, controls, buffers and/or lysis cocktail aspirated may be tracked. For example, a patient sample usage module 650 may track and amount of patient sample used and what assays have been populated on the test plate 225. The patient sample volumes may be displayed graphically. The patient sample usage module 650 may also generate a patient sample usage summary screen 1256S (FIG. 12) that may display the sample IDs and their position on the one or more sample racks 226 (e.g., deck 1-Deck 4), and may also signify whether a sample has received an order or not (e.g., by color). Further, the patient sample usage summary screen 1256S may indicate whether a location on the sample rack 226 is empty.

In accordance with block 418, the extraction plate 220 and its contents may undergo various processing cycles including incubation (including heating and cooling), shaking, multiple washing cycles with addition of various wash buffers (e.g., WB1-WB3 or the like), magnetic separation, and the addition of the elution buffer (EB). The various processing cycles are all known and will not be further expanded on herein. Once processing on the extraction plate 220 has been completed, eluted patient samples and eluted control samples remain in the extraction plate wells of the extraction plate 220. These eluted patient samples and eluted control samples are ready for processing in the next phase of the processing that takes place on the test plate 225 (e.g., PCR test plate).

In block 420, the various test plate wells of the test plate 225, which includes two or more assay regions (e.g., four regions shown in FIG. 3), may be populated with purified samples (referred to as "eluate" herein). The act of populating is designated by arrow 348, wherein one or more robots 235D and one or more coupled pipettes 236D aspirate and dispense the various eluted patient samples and eluted control samples from the extraction plate wells of the extraction plate 220 and transfer these eluate to the various assay regions (e.g., 225A-225D) of the test plate 225. Eluate includes patient sample eluate (e.g., SE1, SE2, SE3, SE4, SE5, SE6, S.E, through SnE) that have been processed through the extraction plate wells of the extraction plate 220, as well as any control sample eluate (e.g., PR1E, PR2E, PR3E, PR4E, UCE) from the various controls (e.g., universal control UC and pre-processed controls PR1-PR4) that have been processed through the extraction plate 220. S.E designates any sample eluate between patient sample eluate S6E and SnE.

The assay regions (e.g., assay regions 225A-225D) may be populated in any order or sequence. For example, the population method may include a first in first out (FIFO) methodology. Where possible, eluate from a single well of the extraction plate 220 should be aspirated and then dispensed before going on to dispense a different eluate. For example, the pre-processed control eluate (e.g., PR1E, PR2E, PR3E, PR4E) may each be aspired and dispensed first into the various assay regions (e.g., assay regions 225A-225D), followed by aspirating and dispensing the universal control eluate (UCE) to the various assay regions (e.g., assay regions 225A-225D). These may be followed by aspirating and dispensing of any post-processed control (e.g., P01, P03), which have not been processed through the extraction plate 220. Finally, the patient sample eluate may be aspired and dispensed into the various assay regions (e.g., assay regions 225A-225D). Other orders of dispensing of the pre-processed controls, post-processed controls, Universal controls and/or sample eluate may be used.

Each one of the patient sample eluate may be aspirated from an individual extraction plate well and then dispensed into multiple test plate wells of the test plate 225 located within the various assay regions according to one aspect. Dispensing may be based on the electronic plate map (e.g., plate map file - *.MAP file)). Thus, a patient sample eluate, based on the determined assay orders, may be dispensed into one assay region only, more than one assay region, or all assay regions. As should be apparent, extreme flexibility in assay population, including full or near full utilization of the test plate 225 may be provided by the SP method 400 and SP apparatus 200.

It should also be apparent that, pre-processed controls may be selected and provided for some, all, or none of the assay regions, as selected by the Tech when running the dynamic assay run setup module 642. Additionally, any post-processed controls (e.g., post-processed controls P01, P03), which are effectively pre-prepared eluate from a kit, may be aspirated and dispensed directly to one or more test plate wells of the respective assay regions (e.g., assay regions 224A, 225B, 225C, and 225D) of the test plate 225, depending upon which assay region has post-processed controls as part of its assay data set. Post-processed controls may be selected and provided for some, all, or none of the assay regions, as selected by the Tech when running the dynamic assay run setup module 642. A time module 655 may instruct the Tech when to add the post-processed controls (e.g., post processed controls P01, P03). Time module 655 may audibly beep and may should a time remaining until the No SP carrier may be loaded. Primer or probe (or primer probe mix) for those protocols desiring primer and probe may be added corresponding assay region. Likewise, enzyme for those protocols desiring enzyme may be added to the corresponding assay region.

The above process blocks of the SP method 400 may be executed or performed in an order or sequence not limited to the order and sequence shown and described. For example, in some embodiments, process block 404 may be performed before or simultaneously with process block 406; process block 410 may be performed before or simultaneously with process block 408; and/or process block 414 may be performed before or simultaneously with process block 412.

As can be seen from FIG. 3, more than one control sample eluate (e.g., PR1E) may be selected and provided in a single assay (e.g., assay region 225A). Also shown is the inclusion of a universal control elate UCE in each region (e.g., assay regions 225A-225D). An assay region may include patient sample eluate only and no controls, patient sample eluate (with or without internal controls) together with various combinations of universal control eluate, pre-processed control eluate, and post-processed controls. Certain controls have been described herein. However, the SP method 400 and SP apparatus 200 may easily incorporate other controls or calibrators either globally or as a selectable part of any assay data set, such as positive controls or independent controls.

Once all the patient sample eluate (e.g., S1E - SnE) and control eluate (UCE, PR1E-PR4E, P01, P03) have been populated into the respective test plate wells of the various assay regions (e.g., assay regions 225A-225D) of the test plate 225 per the electronic plate map, the master mixes (e.g., MM1-MM4) and any other NO SP reagent may be aspirated and dispensed to the respective assay regions (e.g., assay regions 225A-225D) by one or more robots 235C and one or more coupled pipettes 236C, wherein one type of master mix is dedicated to each assay type, and a single assay type is provided in each assay region (e.g., assay regions 225A-225D). Thus, master mix MM1 is dispensed to all test plate wells in the first assay region 225A, master mix MM2 is dispensed to all test plate wells in the second assay region 225A, and so on. Additional reagent dispenses may take place per assay in some embodiments. Once the assay regions (e.g., assay regions 225A-225D) are populated per the electronic plate map, the test plate 225 may be transferred to the AD Device 238.

### EXAMPLE - Dynamic Assay Selection and Sample Preparation

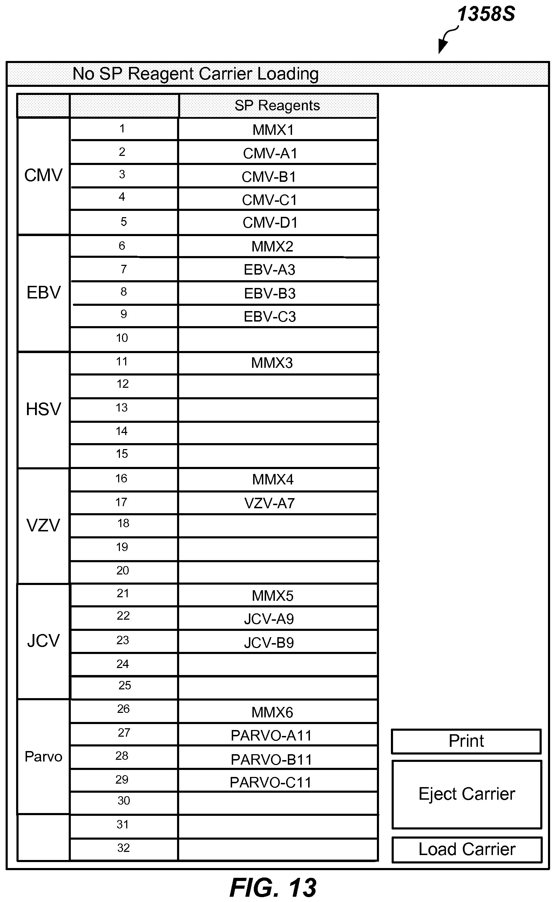

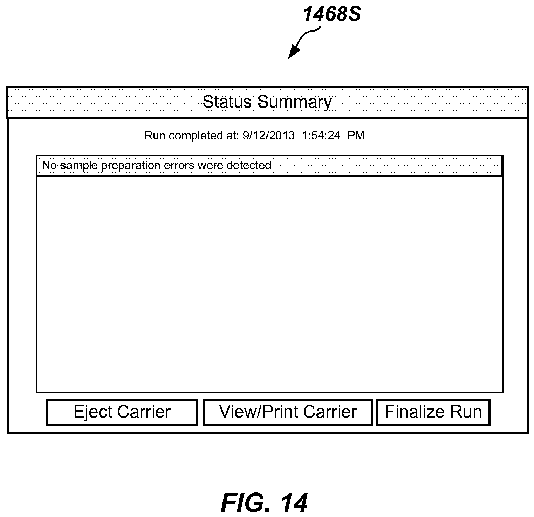

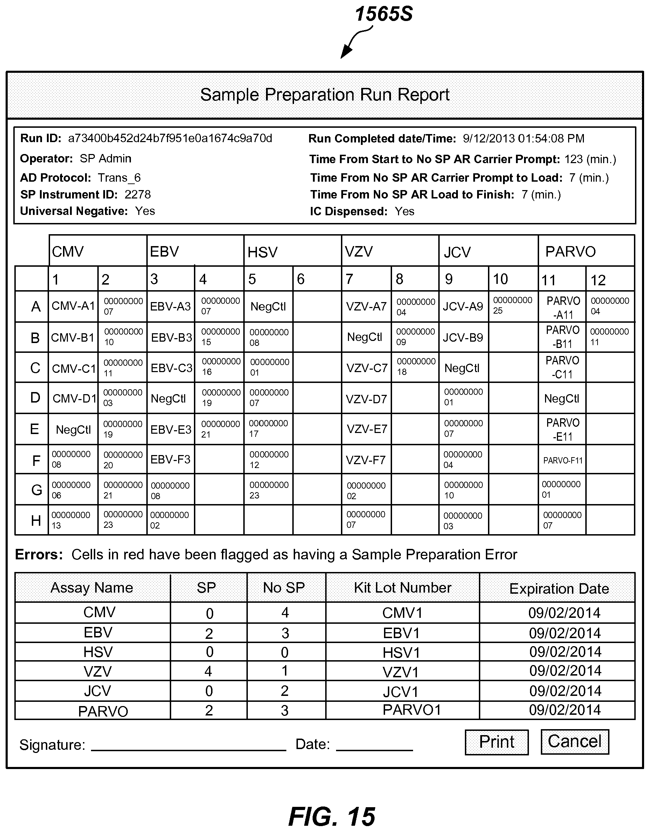

The following provides an outline of a workflow navigation through an example sample preparation (SP) method carried out by the SP apparatus 200 in accordance with one or more embodiments.
**Step 1** - The user (hereinafter "Tech" standing for technician) may place consumables on one or more autoload trays of the SP apparatus 200 (on the SP apparatus 200 but not yet loaded into the interior deck of the SP apparatus 200). The consumables may include:
   1000 ml pipette tips (up to 9 trays)
   300 ml pipette tips (up to 2 trays)
   one or more deep well extraction plates (e.g., 2 deep well extraction plates)
   a PCR micro-well test plate
   Wash buffer 1 (1 trough)
   Wash buffer 2 (1 trough)
   Wash buffer 3 (1 trough)
   Elution buffer (1)
   Lysis buffer (1)
   Magnetic beads (1)
   Proteinase K (PK, 1 tube)
**Step 2** - The Tech may choose a dynamic protocol (see block 404 - FIG. 4) from a Protocol table on the ShellScreen (e.g., workflow module screen 740S) and select "Run Protocol." This selection starts the workflow for the dynamic assay method provided in accordance with embodiments.
**Step 3** - The sample preparation (SP) software may prompt the Tech to empty the solid waste container (optional).
**Step 4** - The Tech may empty the waste container.
**Step 5** - The SP Software displays the dynamic assay run setup screen 842S (Assay Run Setup), which may be as shown in FIG. 8.
**Step 6** - The Tech selects the number of assays to run (see block 406).
**Step 7** - The SP Software enables and disables a number of assay data sets that correspond to the selected number of assays to run from block 406. For example, if 6 options are provided, and four are selected, then two would be disabled. Assay data sets may include fields for: Assay Name, Number of Pre-extract Controls, Number of Post-extract Controls, Kit Lot Code and Expiration Date.
**Step 8** - The Tech defines an assay data set. The assay data set may include the assay name, pre-processing controls, post-processing controls, and may also include lot code and lot expiration date. Note: after the first run, the assays may be selected from the available, previously-defined assay data sets.
**Step 9** - The Tech repeats by returning to **Step 8** until all of selected and enabled assay data sets are populated with desired assay run configurations. Each of the desired assay run configurations may be different, such as shown in FIG. 8.
**Step 10** - The Tech may, in block 410, optionally select whether to dispense internal controls (IC), depending on the type of assays that have been selected. The IC may be a suitable type of IC, and the IC may be selected to be applied to all enabled assays, as is shown in FIG. 8. Optionally, whether to use an IC may be a selectable option for each assay, much like the number of pre-processed controls.
**Step 11** - The Tech may also select whether to use a universal negative control or not, thus combining any universal negative control within the per assay set of pre-processed and/or post processed controls. The Tech may also select the Amplification Detection (AD) Protocol Name that will be used. The AD Protocol Name may be used to link the selected dynamic protocol and the assay data sets to the AD TDef via the electronic plate map (e.g., PCR plate map). AD TDef is defined herein as the test definition file including PCR thermal Cycling conditions, and signal detection parameters.
**Step 12** - Once all assay data sets are completed, the Tech selects Continue on the dynamic assay run setup screen 842S displayed on the display 232 to continue the run.
**Step 13** - The SP Software of the SP method 400 prompts the Tech to load the sample carriers (e.g., sample racks 226) containing the patient samples 228 to prepare or end the run.
**Step 14** - The Tech loads the sample carrier (e.g., sample rack 226) containing the patient samples 228 onto the autoload tray then prompts the SP apparatus 200 to load the patient samples 228.
**Step 15** - The SP software program may pull the sample carrier (e.g., sample rack 226) onto the deck and may read all carrier IDs, sample ID's (e.g., barcodes), and sample slot locations for sample containers 229 (e.g., sample tubes) contained on the sample rack 226. The reading process may be performed by any suitable reader 233.
**Step 16** - The SP software program may determine that there are no barcode read errors.
**Step 17** - The SP software program may check for additional sample carriers (e.g., sample racks 226) and if an unread sample rack is detected, the SP software program may repeat from step 15.
**Step 18** - The SP software program may prompts the Tech to confirm the number of sample containers 229 (e.g., sample tubes) detected in each sample carrier (e.g., in each sample rack 226).
**Step 19** - The Tech confirms that the system (e.g., SP apparatus 200) read the correct number of patient samples (e.g., number of sample containers 229).
**Step 20** - The SP software program may query the LIS 234 for each patient sample 228 to determine what assays will be run for each patient sample 228.
**Step 21** - The LIS (if available, connected, communicating) may respond with the assay orders for the patient sample identifications (sample IDs) associated with each patient sample 228.
**Step 22** - The SP software program may then populates a work list file of the work list and plate map display module 644 with the sample IDs and open assay orders from the LIS 234 based on the sample IDs (e.g., barcodes) found in the sample carrier (e.g., sample rack 226). Any patient samples 228 that don't include orders in LIS 234 may be manually entered. Also, if the LIS is not communicating, the work list file may be manually populated by the Tech.
**Step 23** - The SP software program may display a work list and plate map display screen 944S (otherwise referred to as a "work list editor") on the display 232 showing a graphical representation of the Plate Map (e.g., PCR plate map), and may also show the work list 945.
**Step 24** - The SP software program may populate the PCR Plate Map with the controls, calibrators and sample IDs based on the Dynamic TDef configuration and the Work List selections.
**Step 25** - The Tech may modify the names of the Calibrator/Controls in the plate map to align with what he/she will be loading.
**Step 26 -** The Tech may indicate that the workflow should continue forward by selecting Continue from the work list and plate map display screen 944S.
**Step 27** - The SP software program may audibly (beep) and/or visually prompt the Tech to load all consumables onto one or more autoload trays. Consumables may include, but not limited to, the tips, deep well and test plates, and various Pre-Extract Analyte Reagent (SP reagents) and buffers. The Tech should ensure caps are removed from all tubes and troughs, and then may select load consumables.
**Step 28** - The SP software program may load the Pre-Extract Analyte Reagent carriers (SP Reagent carriers) and then may scan, read and monitor if the correct Pre-Extract Analyte Reagents are loaded. The SP software program then may check the liquid level for each Pre-Extract Analyte Reagent (including PK and IC).
**Step 29** - The SP software program may check to see that the consumable carriers are available on the one or more autoload trays, and may load them onto the deck of the SP apparatus 200.
**Step 30** - The SP software program may include a time module 655 (FIG. 6) which may show a graphical image of a Sample Preparation Process Dialog Time Box counting down the time to the loading of the post-extraction reagents (No SP Reagents, as shown in FIG. 11).
**Step 31a-** The Automated SP Instrument performs a step by step sample preparation on the extraction plate 220 based on work list 945 and plate map 946 (FIG. 9) from Pre-Extract Analyte Reagents up to, but not including, the Post-Extract Analyte Reagents.
**Step 31b** - The SP apparatus 200 pipettes all patient samples from the sample tube carrier(s) (e.g., sample racks 226) to the extraction plate 220 based on the work list 945 and plate map 946.
**Step 31c** - The SP software program may include patient sample usage module 650 (FIG. 6) that may report to the Tech the sample tube usage status, showing the status of each patient sample S1-Sn) but not waiting for a response. A patient sample usage summary screen1256S is shown in FIG. 12. The status of each sample may be presented with multiple colors.
**Step 32** - The SP software program may prompts the Tech to load the post-extract carrier (No SP carrier) onto the autoload tray (e.g., audibly (beeps) and/or visually).
**Step 33** - Once the prompt from the SP software program is recognized and acknowledged (clicked) by the user (see FIG. 13), the pre-extract carrier (SP carrier) is ejected by the SP apparatus 200.
**Step 34** - The Tech removes the pre-extract carrier from the autoload tray and places the post-extract carrier (NO SP carrier) in its place and then prompts the instrument that it is ready to be loaded.
**Step 35** - Upon selecting Load Carrier from the Post extract carrier Loading Screen 1358S (FIG. 13), the SP software program may load the Post-Extract Analyte Reagent carrier (NO SP carrier) onto the deck, and may scan for controls and analyte reagents, and may check that the liquid level of each is as expected.
**Step 36** - The SP software program directs the SP apparatus 200 through the remaining step by step execution of the sample preparation run based on the Dynamic TDef and the electronic plate map. In this step, the extraction plate processing is carried out as discussed above, and then the test plate 225 is populated. Once the SP method 400 is completed, a run complete screen is displayed.
**Step 37** - The SP software program may generate a plate map file (*.MAP file) within a plate map file module 660 based on the electronic plate map and stages it for transmission to the AD Software of the AD device 238 (FIGs. 2 and 6). Transmission may be by Ethernet communication or other suitable means
**Step 38** - The SP software program may generate a Sample Preparation Run Report 1565S as shown in FIG. 15 from an error report module 664, and may stores the report as a file to a Maintenance \ Logfiles folder.
**Step 39** - The SP software program, via a status module 668, may notify the Tech that the sample preparation run has been completed (e.g., audibly (beeps) and/or provides a visual on a status summary screen 1468S as shown in FIG. 14).
**Step 40** - The Tech may then view a sample preparation run report screen1565S (FIG. 15) via the status summary screen 1468S, and may also print that report.
**Step 41** - The Tech may import the plate map file generated by the plate map file module 660, which opens the AD Test Definition on the AD Software of the AD device 238. Note: the MAP file may be located at "C:\Name\PlateMap".
**Step 42** -Via the status summary screen 1468S, the Tech may unload all carriers, including the test plate 225 (PCR plate) from the SP apparatus 200, caps the test plate 225 and exits the screen.
**Step 43** - The Tech loads the test plate 225 (e.g., PCR plate) on the AD device 238.
**Step 44** - The Tech sets up the AD test with appropriate optical channel names which may correspond to the assay names and optionally identifies calibrator/control wells based on the printed Run Report
**Step 45** - The Tech clears/unselects wells from data gathering that correspond to wells with sample preparation errors on the Sample Preparation Run Report printed from the sample preparation run report screen 1565S.
**Step 46** - The Tech initiates a run of the AD device 238.
**Step 47** - The AD Software runs the AD Test Definition, processing the test plate 225 (e.g., PCR plate) to completion.
**Step 48** - The Tech reviews the results versus the Sample Preparation Run Report in order to detect any results that were not cleared/unselected from data gathering due to sample preparation errors. The Tech determines what mitigating action to take if results corresponding to sample preparation errors are detected.
**Step 49** - The Tech sends the test results to the LIS 234, e.g., by opening the LIS interface of the SP software program.
**Step 50** - The scenario ends.

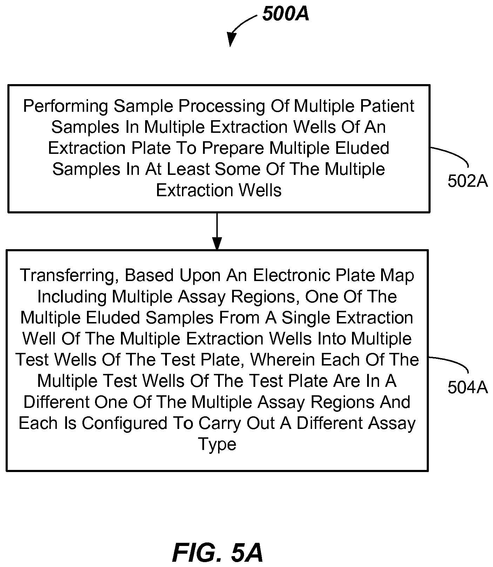

FIG. 5A illustrates a flowchart of a method of populating a test plate (e.g., test plate 225) having multiple test wells according to further embodiments. The method 500A includes, in block 502A, performing sample processing of multiple patient samples (e.g., patient samples S1-Sn) in multiple extraction wells of an extraction plate (e.g., extraction plate 220) to prepare multiple eluted samples (e.g., S1E-SnE) in at least some of the multiple extraction wells, and, in block 504A, transferring, based upon an electronic plate map (see plate map 946 of FIG. 9) including multiple assay regions (e.g., 6 assay regions shown in FIG. 9), one of the multiple eluted samples from a single extraction well of the multiple extraction wells into multiple test wells of the test plate (e.g., test plate 225), wherein each of the multiple test wells of the test plate are in a different one of the multiple assay regions (e.g., 225A-225D) and each is configured to carry out a different assay type (e.g., assay types CMV-PARVO).

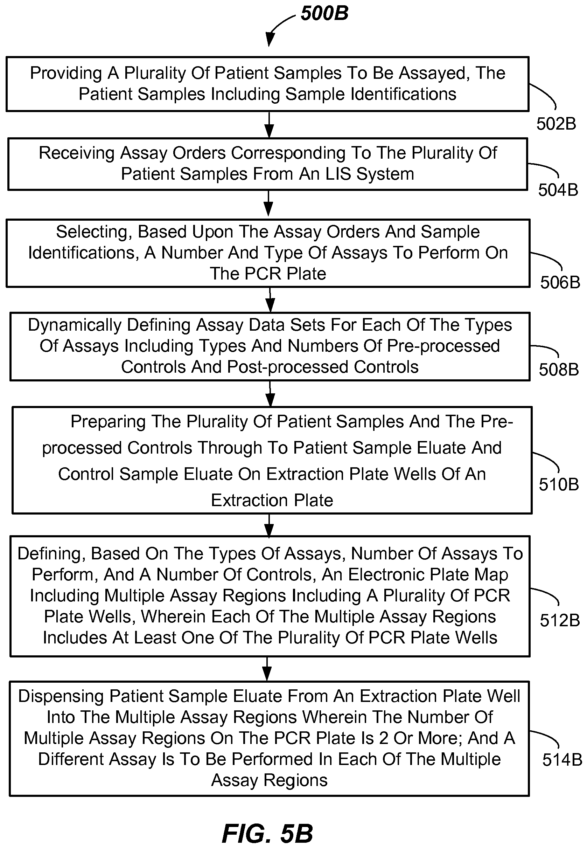

FIG. 5B illustrates a flowchart of a method of preparing samples for assaying on a PCR plate having a plurality of PCR plate wells according to further embodiments. The method 500B includes, in block 502B, providing a plurality of patient samples (e.g., S1-Sn) to be assayed, the patient samples including sample identifications (e.g., barcodes of sample containers 229), and in block 504B, receiving assay orders corresponding to the plurality of patient samples from an LIS (e.g., LIS 234). The method 500B includes, in block 506B, selecting, based upon the assay orders and sample identifications, a number and type of assays to perform on the PCR plate, and, in block 508B, dynamically defining assay data sets for each of the types of assays including at least types and numbers of pre-processed controls and post-processed controls. The method 500B includes, in block 510B, preparing the plurality of patient samples and the pre-processed controls through to patient sample eluate and control sample eluate on extraction plate wells of an extraction plate (e.g., extraction plate 220). The method 500B includes, in block 512B, defining, based on the types of assays, number of assays to perform, and a number of controls, an electronic plate map (e.g., see plate map 946 of FIG. 9) including multiple assay regions including a plurality of PCR plate wells, wherein each of the multiple assay regions (e.g., assay regions 225A-225D) includes at least one of the plurality of PCR plate wells, and, in block 514B, dispensing patient sample eluate from an extraction plate well into the multiple assay regions wherein the number of multiple assay regions on the PCR plate (e.g., test plate 225) is 2 or more; and a different assay is to be performed in each of the multiple assay regions.

Some embodiments, or portions thereof, may be provided as a computer program product or software that may include a machine-readable medium having non-transient instructions stored thereon, which may be used to program the controller 230, to perform a sample preparation method in accordance with one or more embodiments. In one embodiment, a machine-readable medium having non-transient instructions stored thereon may be provided. The "machine-readable medium" refers to any statutory medium that participates in providing data (*e.g.,* instructions) that may be read by a computer, a processor, or like device. Such a medium may take many forms, including but not limited to non-volatile media, volatile media, and specific statutory types of transmission media. Non-volatile media include, for example, optical or magnetic disks, and other persistent memory. Volatile media include DRAM, which typically constitutes the main memory. Common forms of computer-readable media include, for example, a floppy disk, a flexible disk, hard disk, magnetic tape, any other magnetic medium, a CD-ROM, Digital Video Disc (DVD), any other optical medium, punch cards, paper tape, any other physical medium with patterns of holes, a RAM, a PROM, an EPROM, a FLASH-EEPROM, a USB memory stick, a dongle, any other memory chip or cartridge, a carrier wave, or any other medium from which a computer can read. The terms "computer-readable memory" and / or "tangible media" specifically exclude signals, waves, and wave forms or other intangible or transitory media that may nevertheless be readable by a computer.

The machine-readable medium having non-transient instructions includes a dynamic run setup module configured to define a plurality of assay data sets with desired run configurations based at least in part on a number and type of assays to run, and number and type of controls; and a work list and plate map display module configured to generate an electronic plate map including spatial information correlated to locations (e.g., test plate well locations) within multiple assay regions of a test plate of patient sample eluate and control eluate and optionally one or more post-processed controls.

The sample preparation software program may further include additional modules that are adapted to carry out specific programming tasks, such as workflow start module 640, patient sample usage module 650, required inventory module 652, status module 668, and error report module 664, as discussed above.

### DEFINITIONS

**Display** - an area or device that conveys information to a viewer. The information may be dynamic, in which case, a liquid crystal display (LCD), light emitting diode (LED), cathode ray tube (CRT), Digital Light Processing (DLP), plasma, rear projection, front projection, or the like may be used to form the display. The aspect ratio of the display may be 4:3, 16:9, or the like. Furthermore, the resolution of the display may be any appropriate resolution such as 480i, 480p, 720p, 1080i, 1080p, or the like. The format of information sent to the display may be any appropriate format such as Standard Definition Television (SDTV), Enhanced Definition TV (EDTV), High Definition TV (HDTV), or the like. Some displays may be interactive and may include touch screen features or associated keypads as is well understood.
**Eluate** - a substance (e.g., a target nucleic acid) separated out by, or the product of, elution or elutriation.
**Pre-processed controls** - Those process controls that have been processed on the extraction plate along with the patient samples and then are transferred to the PCR plate.
**Post-processed controls** - Those process controls that have been processed by the manufacturer and get directly loaded onto to the test plate (e.g., PCR plate) along with eluted samples and master mix.
**Internal controls** - Those process controls that are added to the patient samples on the extraction plate that indicate that the process has proceeded without any reaction issues that interfere with the end result.
**Proteinase K** - Proteinase K is a broad-spectrum serine protease. Proteinase K is commonly used in molecular biology to digest protein and remove contamination from preparations of nucleic acid. Addition of Proteinase K to nucleic acid preparations rapidly inactivates nucleases that might otherwise degrade the DNA or RNA during purification.
**Master mix** - Master mix is premixed, ready-to-use solution containing polymerase components and other components (e.g., Taq DNA polymerase, dNTPs, MgCl₂ and reaction buffers) at optimal concentrations for efficient amplification of DNA templates.

The foregoing description discloses only example embodiments of the invention. Modifications of the above-disclosed apparatus, systems, and methods which fall within the scope of the invention will be readily apparent to those of ordinary skill in the art. Accordingly, while the present invention has been disclosed in connection with example embodiments, it should be understood that other embodiments may fall within the scope of the invention, as defined by the following claims.

## Claims

1. A method of preparing samples for assaying on a test plate having a plurality of wells, comprising:
receiving, via a user interface, instructions to run a first assay type and a second assay type;
determining sample identifications for a plurality of samples to be assayed;
receiving assay orders corresponding to the sample identifications;
defining, based on the assay orders and sample identifications, first and second assay regions of the plurality of wells, wherein each defined assay region includes at least one of the plurality of wells; and
creating an electronic plate map including a graphical representation of the test plate indicating the first assay region and the second assay region and instructions to run a first assay type on samples contained within the first assay region, and to run a second assay type on samples contained within the second assay region, wherein the electronic plate map includes and defines two or more assay regions, which are each a collection of physical well locations on the test plate.

2. The method of claim 1, comprising three or more assay regions, and wherein a different assay type is prepared in each of the three or more assay regions.

3. The method of claim 2, comprising displaying a graphical representation of the test plate indicating the three or more assay regions.

4. The method of claim 1, comprising:
simultaneously including
one or more pre-processed control sample eluate from an extraction plate on one or more assay regions of the test plate, and
one or more post-processed control on one or more assay regions of the test plate.

5. The method of claim 1, comprising defining assay data sets for each of the first assay type and the second assay type being run in a particular batch, the assay data sets including at least an assay name, and a presence of pre-processed controls, a presence of post-processed controls, or both.

6. The method of claim 1, comprising displaying a graphical representation of at least some inventory requirements used to prepare the assays to be run on the test plate including the first assay region and the second assay region.

7. The method of claim 1, comprising defining the first and second assay regions based on the assay orders, the sample identifications, and controls.

8. The method of claim 1, comprising simultaneously including one or more pre-processed control samples and one or more post-processed control samples on the test plate.

9. The method of claim 1, comprising providing a list of at least some inventory requirements for processing of the first and second assays and the samples.

10. The method of claim 1, comprising generating an inventory image file based upon the electronic plate map.

11. The method of claim 1 further comprising transferring the electronic plate map to an amplification and detection device.

12. The method of claim 11, wherein the electronic plate map includes instructions to run a first assay type on eluted patient samples and a first master mix contained within the first assay region, and to run a second assay type on eluted patient samples and a second master mix contained within the second assay region.

13. The method of claim 1 comprising:
performing sample processing of multiple patient samples in multiple extraction plate wells of an extraction plate to prepare multiple eluted samples in at least some of the multiple extraction plate wells; and
transferring, based upon the electronic plate map including multiple assay regions, one of the multiple eluted samples from a single extraction plate well of the multiple extraction plate wells into multiple test wells of the test plate, wherein each of said multiple test wells of the test plate are in a different one of the multiple assay regions and each is configured to carry out a different assay type.

14. The method of claim 13, wherein the one of the multiple eluted samples from the single extraction well of the multiple wells is transferred to three or more of the multiple test wells of the test plate, wherein each of the three or more of the multiple test wells is in a different plate region.

15. The method of claim 13, wherein the one of the multiple eluted samples is transferred to between two and six of the multiple test wells of a test plate.

16. The method of claim 13, wherein each of the different assay types is provided in a different assay region of the test plate.

17. The method according to claim 1 wherein the test plate is a PCR plate having a plurality of PCR plate wells, comprising:
providing a plurality of patient samples to be assayed, the patient samples including sample identifications;
receiving assay orders corresponding to the plurality of patient samples from a Laboratory Information System (LIS); selecting, based upon the assay orders and sample identifications, a number and type of assays to perform on the PCR plate;
dynamically defining assay data sets for each of the types of assays including types and numbers of pre-processed controls and post-processed controls;
preparing the plurality of patient samples and the pre-processed controls through to patient sample eluate and control sample eluate on extraction plate wells of an extraction plate;
defining, based on the types of assays, number of assays to perform, and a number of controls, the electronic plate map including multiple assay regions including a plurality of PCR plate wells, wherein each of the multiple assay regions includes at least one of the plurality of PCR plate wells; and
dispensing patient sample eluate from an extraction plate well into the multiple assay regions wherein a number of the multiple assay regions on the PCR plate is two or more; and a different assay is to be performed in each of the multiple assay regions.

18. A sample preparation apparatus, comprising:
an extraction plate;
a test plate;
a memory operative to store at least patient sample identifications, assay orders for each patient sample, and number and type of assays;
a processor coupled to the memory and operable to generate an electronic plate map including a graphical representation of the test plate indicating the multiple assay regions, which are each a collection of physical well locations on the test plate, based at least on a number of the patient samples, a number and type of assay orders for each of the patient samples, and number of controls; and
one or more robots each including one or more coupled pipettes configured to transfer patient sample eluate from an extraction plate well of the extraction plate and master mix to the multiple assay regions.

19. The sample preparation apparatus of claim 18, comprising a communication device configured to transfer an electronic data file including information from the electronic plate map to an amplification and detection device.

20. The sample preparation apparatus of claim 18, comprising sample preparation software including a required inventory module configured to be executed by the processor to prepare an image file or list of at least some of the inventory requirements for the multiple assays.

21. A machine-readable medium having non-transient instructions stored thereon for performing a method of claims 1-17, comprising:
a dynamic run setup module configured to define a plurality of assay data sets with desired run configurations based at least in part on a number and type of assays to run, and number and type of controls; and
a work list and plate map display module configured to generate an electronic plate map including a graphical representation indicating multiple assay regions of a test plate of patient sample eluate and control eluate and optionally one or more post-processed controls.

## Patentansprüche

1. Verfahren zur Vorbereitung von Proben zum Testen auf einer Testplatte mit mehreren Löchern, umfassend:
Empfangen, über eine Benutzerschnittstelle, von Anweisungen zum Durchführen eines ersten Testtyps und eines zweiten Testtyps;
Bestimmen von Probenkennungen für mehrere zu testende Proben;
Empfangen von Testaufträgen, die den Probenkennungen entsprechen;
Definieren, basierend auf den Testaufträgen und den Probenkennungen, einer ersten und einer zweiten Testregion der mehreren Löcher, wobei jede definierte Testregion wenigstens eines der mehreren Löcher aufweist; und
Erstellen einer elektronischen Plattenübersicht, die eine grafische Darstellung der Testplatte, welche die erste Testregion und die zweite Testregion ausweist, und Anweisungen zum Durchführen eines ersten Testtyps an Proben, die in der ersten Testregion enthalten sind, und zum Durchführen eines zweiten Testtyps an Proben, die in der zweiten Testregion enthalten sind, beinhaltet, wobei die elektronische Plattenübersicht zwei oder mehr Testregionen aufweist und definiert, die jeweils eine Gruppe physischer Lochpositionen auf der Testplatte darstellen.

2. Verfahren nach Anspruch 1, das drei oder mehr Testregionen umfasst und bei dem in jeder der drei oder mehr Testregionen ein anderer Testtyp vorbereitet wird.

3. Verfahren nach Anspruch 2, das das Anzeigen einer grafischen Darstellung der Testplatte umfasst, die die drei oder mehr Testregionen ausweist.

4. Verfahren nach Anspruch 1, umfassend:
gleichzeitig einzuschließen:
ein oder mehrere vorverarbeitete Kontrollprobeneluate von einer Extraktionsplatte in einer oder mehreren Testregionen der Testplatte, und
eine oder mehrere nachverarbeitete Kontrollen in einer oder mehreren Testregionen der Testplatte.

5. Verfahren nach Anspruch 1, umfassend das Definieren von Testdatensätzen für sowohl den ersten Testtyp als auch den zweiten Testtyp, die in einer bestimmten Reihe durchgeführt werden, wobei die Testdatensätze wenigstens einen Testnamen und ein Vorhandensein vorverarbeiteter Kontrollen, ein Vorhandensein nachverarbeiteter Kontrollen oder beides beinhalten.

6. Verfahren nach Anspruch 1, umfassend das Anzeigen einer grafischen Darstellung von wenigstens einigen Inventaranforderungen, die zur Vorbereitung der an der Testplatte durchzuführenden Tests dienen, einschließend die erste Testregion und die zweite Testregion.

7. Verfahren nach Anspruch 1, umfassend das Definieren der ersten und der zweiten Testregion basierend auf den Testaufträgen, den Probenkennungen und Kontrollen.

8. Verfahren nach Anspruch 1, umfassend, gleichzeitig eine oder mehrere vorverarbeitete Kontrollproben sowie eine oder mehrere nachverarbeitete Kontrollproben auf der Testplatte einzuschließen.

9. Verfahren nach Anspruch 1, umfassend das Bereitstellen einer Liste von wenigstens einigen Inventaranforderungen zum Verarbeiten des ersten und des zweiten Tests und der Proben.

10. Verfahren nach Anspruch 1, umfassend das Erzeugen einer Inventarbilddatei basierend auf der elektronischen Plattenübersicht.

11. Verfahren nach Anspruch 1, ferner umfassend das Übertragen der elektronischen Plattenübersicht an eine Verstärkungs- und Detektionsvorrichtung.

12. Verfahren nach Anspruch 11, wobei die elektronische Plattenübersicht Anweisungen beinhaltet, einen ersten Testtyp an eluierten Patientenproben und einer ersten Mastermischung, die in der ersten Testregion enthalten sind, durchzuführen und einen zweiten Testtyp an eluierten Patientenproben und einer zweiten Mastermischung, die in der zweiten Testregion enthalten sind, durchzuführen.

13. Verfahren nach Anspruch 1, umfassend:
Durchführen einer Probenverarbeitung mehrerer Patientenproben in mehreren Extraktionsplattenlöchern einer Extraktionsplatte, um mehrere eluierte Proben in wenigstens einigen der mehreren Extraktionsplattenlöcher vorzubereiten; und
Überführen, basierend auf der elektronischen Plattenübersicht, die mehrere Testregionen aufweist, einer der mehreren eluierten Proben aus einem einzelnen Extraktionsplattenloch der mehreren Extraktionsplattenlöcher in mehrere Testlöcher der Testplatte, wobei jedes der mehreren Testlöcher der Testplatte in einer anderen der mehreren Testregionen liegt und jeweils dafür ausgelegt ist, einen anderen Testtyp durchzuführen.

14. Verfahren nach Anspruch 13, wobei die eine der mehreren eluierten Proben aus dem einzelnen Extraktionsloch der mehreren Löcher in drei oder mehr der mehreren Testlöcher der Testplatte überführt werden, wobei jedes der drei oder mehr der mehreren Testlöcher in einer anderen Plattenregion liegt.

15. Verfahren nach Anspruch 13, wobei die eine der mehreren eluierten Proben auf zwischen zwei und sechs der mehreren Testlöcher einer Testplatte überführt wird.

16. Verfahren nach Anspruch 13, wobei die verschiedenen Testtypen jeweils in einer anderen Testregion der Testplatte vorgesehen sind.

17. Verfahren gemäß Anspruch 1, wobei die Testplatte eine PCR-Platte mit mehreren PCR-Plattenlöchern ist, umfassend:
Bereitstellen mehrerer zu testender Patientenproben, wobei die Patientenproben Probenkennungen aufweisen;
Empfangen von Testaufträgen, die den mehreren Patientenproben entsprechen, von einem Laborinformationssystem (LIS);
Auswählen, basierend auf den Testaufträgen und den Probenkennungen, einer Anzahl und eines Typs von Tests, die an der PCR-Platte durchgeführt werden sollen;
dynamisches Definieren von Testdatensätzen für jeden der Testtypen einschließlich Typen und Anzahl vorverarbeiteter Kontrollen und nachverarbeiteter Kontrollen;
Vorbereiten der mehreren Patientenproben und der vorverarbeiteten Kontrollen bis hin zum Patientenprobeneluat und Kontrollprobeneluat in Extraktionsplattenlöchern einer Extraktionsplatte;
Definieren, basierend auf den Testtypen, einer Anzahl von durchzuführenden Tests und einer Anzahl von Kontrollen, der elektronischen Plattenübersicht, welche mehrere Testregionen aufweist, die mehrere PCR-Plattenlöcher aufweisen, wobei jede der mehreren Testregionen wenigstens eines der mehreren PCR-Plattenlöcher aufweist; und
Verteilen des Patientenprobeneluats aus einem Extraktionsplattenloch in die mehreren Testregionen, wobei eine Anzahl der mehreren Testregionen auf der PCR-Platte zwei oder mehr beträgt; und wobei in jeder der mehreren Testregionen ein anderer Test durchgeführt wird.

18. Probenvorbereitungsvorrichtung, umfassend:
eine Extraktionsplatte;
eine Testplatte;
einen Speicher, der dazu dient, wenigstens Patientenprobenkennungen, Testaufträge für jede Patientenprobe und Anzahl und Typ der Tests zu speichern;
einen Prozessor, der mit dem Speicher verbunden ist und dazu dient, eine elektronische Plattenübersicht zu erzeugen, die eine grafische Darstellung der Testplatte aufweist, welche die mehreren Testregionen ausweist, die jeweils eine Gruppe physischer Lochpositionen auf der Testplatte bilden, basierend wenigstens auf einer Anzahl der Patientenproben, einer Anzahl und einem Typ der Testaufträge für jede der Patientenproben und der Anzahl der Kontrollen; und
einen oder mehrere Roboter, die jeweils eine oder mehrere gekoppelte Pipetten aufweisen, welche dafür ausgelegt sind, Patientenprobeneluat aus einem Extraktionsplattenloch der Extraktionsplatte und eine Mastermischung in die mehreren Testregionen zu überführen.

19. Probenvorbereitungsvorrichtung nach Anspruch 18, umfassend eine Kommunikationsvorrichtung, die dafür ausgelegt ist, eine elektronische Datendatei, welche Informationen aus der elektronischen Plattenübersicht beinhaltet, an eine Verstärkungs- und Detektionsvorrichtung zu übertragen.

20. Probenvorbereitungsvorrichtung nach Anspruch 18, umfassend Probenvorbereitungs-Software, die ein Modul erforderlichen Inventars beinhaltet, das dafür ausgelegt ist, vom Prozessor ausgeführt zu werden, um eine Bilddatei oder eine Liste von wenigstens einigen der Inventaranforderungen für die mehreren Tests vorzubereiten.

21. Maschinenlesbares Medium mit darauf gespeicherten nichttransienten Anweisungen zum Durchführen eines Verfahrens nach Anspruch 1-17,
umfassend:
ein dynamisches Ablaufeinrichtungsmodul, das dafür ausgelegt ist, mehrere Testdatensätze mit gewünschten Ablaufkonfigurationen zu definieren, wenigstens teilweise basierend auf einer Anzahl und einem Typ der auszuführenden Tests sowie einer Anzahl und einem Typ von Kontrollen; und
ein Anzeigemodul für Arbeitsliste und Plattenübersicht, das dafür ausgelegt ist, eine elektronische Plattenübersicht zu erzeugen, die eine grafische Darstellung aufweist, welche mehrere Testregionen einer Testplatte mit Patientenprobeneluat und Kontrolleluat sowie wahlweise einer oder mehreren nachverarbeiteten Kontrollen ausweist.

## Revendications

1. Procédé de préparation d'échantillons pour un dosage sur une plaque d'essai ayant une pluralité de puits, comprenant :
la réception, via une interface utilisateur, d'instructions pour exécuter un premier type de dosage et un second type de dosage ;
la détermination d'identifications d'échantillons pour une pluralité d'échantillons à doser ;
la réception d'ordres de dosage correspondant aux identifications d'échantillons ;
la définition, sur la base des ordres de dosage et des identifications d'échantillons, de première et seconde régions de dosage de la pluralité de puits, dans lequel chaque région de dosage définie comporte au moins l'un de la pluralité de puits ; et
la création d'une carte de plaque électronique comportant une représentation graphique de la plaque d'essai indiquant la première région de dosage et la seconde région de dosage et des instructions pour exécuter un premier type de dosage sur des échantillons contenus dans la première région de dosage, et pour exécuter un second type de dosage sur des échantillons contenus dans la seconde région de dosage, dans lequel la carte de plaque électronique comporte et définit deux régions de dosage ou plus, qui sont chacune une collection d'emplacements de puits physique sur la plaque d'essai.

2. Procédé selon la revendication 1, comprenant trois régions de dosage ou plus, et dans lequel un type de dosage différent est préparé dans chacune des trois régions de dosage ou plus.

3. Procédé selon la revendication 2, comprenant l'affichage d'une représentation graphique de la plaque d'essai indiquant les trois régions de dosage ou plus.

4. Procédé selon la revendication 1, comprenant :
le fait d'inclure simultanément
un ou plusieurs éluats d'échantillons témoins pré-traités provenant d'une plaque d'extraction sur une ou plusieurs régions de dosage de la plaque d'essai, et
un ou plusieurs témoins post-traités sur une ou plusieurs régions de dosage de la plaque d'essai.

5. Procédé selon la revendication 1, comprenant la définition d'ensembles de données de dosage pour chacun du premier type de dosage et du second type de dosage exécutés dans un lot particulier, les ensembles de données de dosage comportant au moins un nom de dosage, et une présence de témoins pré-traités, une présence de témoins post-traités, ou les deux.

6. Procédé selon la revendication 1, comprenant l'affichage d'une représentation graphique d'au moins certaines exigences inventaires utilisées pour préparer les dosages à exécuter sur la plaque d'essai comportant la première région de dosage et la seconde région de dosage.

7. Procédé selon la revendication 1, comprenant la définition des première et seconde régions de dosage sur la base des ordres de dosage, des identifications d'échantillons, et des témoins.

8. Procédé selon la revendication 1, comprenant le fait d'inclure simultanément un ou plusieurs échantillons témoins pré-traités et un ou plusieurs échantillons témoins post-traités sur la plaque d'essai.

9. Procédé selon la revendication 1, comprenant la fourniture d'une liste d'au moins certaines exigences d'inventaire pour traiter les premier et second dosages et les échantillons.

10. Procédé selon la revendication 1, comprenant la génération d'un fichier d'image d'inventaire sur la base de la carte de plaque électronique.

11. Procédé selon la revendication 1 comprenant en outre le transfert de la carte de plaque électronique à un dispositif d'amplification et de détection.

12. Procédé selon la revendication 11, dans lequel la carte de plaque électronique comporte des instructions pour exécuter un premier type de dosage sur des échantillons de patient élués et un premier mélange maître contenu dans la première région de dosage, et pour exécuter un second type de dosage sur des échantillons de patient élués et un second mélange maître contenu dans la seconde région de dosage.

13. Procédé selon la revendication 1 comprenant :
la réalisation d'un traitement d'échantillon de multiples échantillons de patient dans de multiples puits de plaque d'extraction d'une plaque d'extraction pour préparer de multiples échantillons élués dans au moins certains des multiples puits de plaque d'extraction ; et
le transfert, sur la base de la carte de plaque électronique comportant de multiples régions de dosage, de l'un des multiples échantillons élués provenant d'un puits de plaque d'extraction unique des multiples puits de plaque d'extraction dans de multiples puits d'essai de la plaque d'essai, dans lequel chacun desdits multiples puits d'essai de la plaque d'essai est dans une région différente des multiples régions de dosage et chacun est configuré pour réaliser un type de dosage différent.

14. Procédé selon la revendication 13, dans lequel l'échantillon des multiples échantillons élués provenant du puits d'extraction unique des multiples puits est transféré à trois ou plus des multiples puits d'essai de la plaque d'essai, dans lequel chacun des trois ou plus des multiples puits d'essai est dans une région de plaque différente.

15. Procédé selon la revendication 13, dans lequel l'échantillon des multiples échantillons élués est transféré à entre deux et six des multiples puits d'essai d'une plaque d'essai.

16. Procédé selon la revendication 13 dans lequel chacun des types de dosage différents est prévu dans une région de dosage différente de la plaque d'essai.

17. Procédé selon la revendication 1, dans lequel la plaque d'essai est une plaque PCR ayant une pluralité de puits de plaque PCR, comprenant :
la fourniture d'une pluralité d'échantillons de patient à doser, les échantillons de patient comportant des identifications d'échantillons ;
la réception d'ordres de dosage correspondant à la pluralité d'échantillons de patient en provenance d'un système d'informations de laboratoire (SIL) ;
la sélection, sur la base des ordres de dosage et des identifications d'échantillons, d'un nombre et d'un type de dosages à réaliser sur la plaque PCR ;
la définition dynamique d'ensembles de données de dosage pour chacun des types de dosages comportant des types et des numéros de témoins pré-traités et de témoins post-traités ;
la préparation de la pluralité d'échantillons de patient et des témoins pré-traités par le biais d'un éluat d'échantillon de patient et d'un éluat d'échantillon témoin sur des puits de plaque d'extraction d'une plaque d'extraction ;
la définition, sur la base des types de dosages, d'un nombre de dosages à réaliser, et d'un nombre de témoins, la carte de plaque électronique comportant de multiples régions de dosage comportant une pluralité de puits de plaque PCR, dans lequel chacune des multiples régions de dosage comporte au moins l'un de la pluralité de puits de plaque PCR ; et
la distribution d'un éluat d'échantillon de patient provenant d'un puits de plaque d'extraction dans les multiples régions de dosage dans lequel un nombre des multiples régions de dosage sur la plaque PCR est de deux ou plus ; et un dosage différent doit être réalisé dans chacune des multiples régions de dosage.

18. Appareil de préparation d'échantillon, comprenant :
une plaque d'extraction ;
une plaque d'essai ;
une mémoire opérationnelle pour stocker au moins des identifications d'échantillons de patient, des ordres de dosage pour chaque échantillon de patient, et un nombre et un type de dosages ;
un processeur couplé à la mémoire et opérationnel pour générer une carte de plaque électronique comportant une représentation graphique de la plaque d'essai indiquant les multiples régions de dosage, qui sont chacune une collection d'emplacements de puits physique sur la plaque d'essai, sur la base d'au moins un nombre des échantillons de patient, un nombre et un type d'ordres de dosage pour chacun des échantillons de patient, et un nombre de témoins ; et
un ou plusieurs robots comportant chacun une ou plusieurs pipettes couplées configurées pour transférer un éluat d'échantillon de patient provenant d'un puits de plaque d'extraction de la plaque d'extraction et un mélange maître aux multiples régions de dosage.

19. Appareil de préparation d'échantillon selon la revendication 18, comprenant un dispositif de communication configuré pour transférer un fichier de données électronique comportant des informations de la carte de plaque électronique à un dispositif d'amplification et de détection.

20. Appareil de préparation d'échantillon selon la revendication 18, comprenant un logiciel de préparation d'échantillon comportant un module d'inventaire requis configuré pour être exécuté par le processeur pour préparer une liste ou un fichier d'image d'au moins certaines des exigences inventaires pour les multiples dosages.

21. Support lisible par machine sur lequel sont stockées des instructions non transitoires pour réaliser le procédé des revendications 1 à 17, comprenant :
un module d'organisation d'exécution dynamique configuré pour définir une pluralité d'ensembles de données de dosage avec des configurations d'exécution souhaitées sur la base d'au moins en partie un nombre et un type de dosages à exécuter, et un nombre et un type de témoins ; et
un module d'affichage de liste de travail et de carte de plaque configuré pour générer une carte de plaque électronique comportant une représentation graphique indiquant des multiples régions de dosage d'une plaque d'essai d'un éluat d'échantillon de patient et d'un éluat témoin et facultativement un ou plusieurs témoins post-traités.
